# EUROPÄISCHE PATENTSCHRIFT

(11) **EP 2 993 164 B1**
(45) Veröffentlichungstag und Bekanntmachung des Hinweises auf die Patenterteilung: **15.02.2017**
(21) Anmeldenummer: 14183918.3
(22) Anmeldetag: 08.09.2014
(51) Int. Cl.: C07C 7/20, C08F 2/40

(54) **Phenolische (Thio)Acetale als Inhibitoren der Polymerisation von olefinisch ungesättigten Monomeren**
Phenolic (thio) acetals as inhibitors of the polymerisation of olefinically unsaturated monomers
(Thio)acétales phénoliques comme inhibiteurs de la polymérisation de monomères oléfiniques non saturés

(43) Veröffentlichungstag der Anmeldung: 09.03.2016
(73) Patentinhaber: Evonik Degussa GmbH, 45128 Essen (DE)
(72) Erfinder: Nissen, Felix, 48301 Nottuln (DE); Rinker, Stefanie, 46284 Dorsten (DE); James, Phillip R., SA70 7LE Tenby (GB); Erpeldinger, Oliver, 42489 Wülfrath (DE); Neumann, Manfred, 45770 Marl (DE); Kraushaar, Frank, 45239 Essen (DE)

(56) Entgegenhaltungen:
- US-A- 4 040 911
- US-A- 4 151 211
- US-A- 5 322 960
- KURBASHOV V ET AL: "The inhibition of oxidative polymerization of styrene by phenols. Autovibrational mode", POLYMER SCIENCE U.S.S.R, PERGAMON PRESS, OXFORD, Bd. 26, Nr. 3, 1. Januar 1984 (1984-01-01) , Seiten 604-610, XP025463594, ISSN: 0032-3950, DOI: 10.1016/0032-3950(84)90396-4 [gefunden am 1984-01-01]

## Beschreibung

Diese Erfindung betrifft ein Verfahren zur Inhibierung der Polymerisation von mindestens einem olefinisch ungesättigten Monomer mit mindestens einem phenolischen (Thio)Acetal. Diese Erfindung betrifft außerdem eine Zusammensetzung umfassend mindestens ein olefinisch ungesättigtes Monomer und mindestens ein phenolisches (Thio)Acetal in einem bestimmten Gewichtsverhältnis.

### Hintergrund der Erfindung

Während der Herstellung von olefinisch ungesättigten Monomeren, wie beispielsweise Ethen, Butadien, Isopren, Vinylacetat, (Meth)Acrylsäure, (Meth)Acrylat, Acrolein, Acrylnitril oder Vinyl-substituierten Aromaten, werden diese olefinisch ungesättigten Monomere mehreren Verfahrensschritten unterzogen, wie beispielsweise Destillation oder Extraktion, um unerwünschte Nebenprodukte oder Verunreinigungen zu entfernen. Insbesondere die Produktions- und Destillationsverfahrensschritte werden bei erhöhten Temperaturen durchgeführt.

Olefinisch ungesättigte Monomere neigen jedoch bereits während des Herstellungs- und/oder Reinigungsprozesses zur ungewollten Polymerisation. Die Gefahr einer Polymerisation besteht bei allen oben genannten Monomeren, und dies besonders bei erhöhter Temperatur. Einige dieser olefinisch ungesättigten Monomere, wie beispielsweise Butadien, neigen aber auch bereits während der Lagerung oder beim Transport zu einer spontanen, meist stark exothermen und daher gefährlichen Polymerisation.

Aber auch die eher schleichende Polymerisation olefinisch ungesättigter Monomere während der Produktion und Aufreinigung ist unerwünscht. Zum einen kommt es zu Ablagerungen der Polymere in den Reaktoren und Kolonnen, zum anderen zu einer Mengenreduktion an verfügbaren Monomeren. Ablagerungen des Polymers können unter anderem zu einer verminderten Wärmeübertragung in einzelnen Anlagenbauteilen, und somit zu einer verringerten Produktivität führen.

Des Weiteren können Anlagenbauteile, wie beispielsweise Filter, mit dem unerwünschten Polymer überzogen werden und verstopfen. Dies hat außerplanmäßige Unterbrechungen der Produktion zur Folge, um eine Reinigung der Anlage durchführen zu können. Jeder Ausfall bringt zum einen Reparatur- und Reinigungskosten mit sich, zum anderen bewirkt ein Stillstand auch einen Produktionsausfall. Um diesem Problem zu begegnen, werden den olefinisch ungesättigten Monomeren in der Regel bereits während des Herstellungsverfahrens Additive zugesetzt, die die unerwünschte Polymerisation der olefinisch ungesättigten Monomere verhindern oder zumindest verlangsamen. Solche Additive werden als "Polymerisationsinhibitoren" bezeichnet.

Im Stand der Technik ist eine Vielzahl solcher Polymerisationsinhibitoren beschrieben. Dazu gehören etwa 2,2,6,6-Tetramethylpiperidin-*N*-oxyl und dessen Derivate, Nitroaromaten, wie zum Beispiel 2,4-Dinitro-6-sec-butylphenol, 2,4-Dinitrophenol oder 4,6-Dinitro-*ortho*-kresol, oder andere Additive wie *C*- und/oder *N*-Nitroso-Verbindungen, Hydroxylamine und Oxime.

Eine weitere Klasse von Inhibitoren stellen die Chinonmethide dar. Die Verwendung dieser Substanzklasse zur Polymerisationsinhibierung von Styrol beschreiben Bacha et al. in US 4,003,800 A und in US 4,040,911 A.

Chinonmethide können auch in Kombination mit bekannten Additiven eingesetzt werden, wie zum Beispiel mit Hydroxylaminen (US 2005/0027150 A1), 4-*tert*-Butylkatechol (US 2005/0027150 A1), einer Kombination aus Hydroxylaminen und Katecholen (US 2004/0034247 A1), Nitroxylradikalen (WO 01/40404 A1; WO 02/33026 A1), Nitrosoverbindungen (US 2006/0283699 A1) oder einer Kombination aus *C*-Nitrosoanilinen und Chinoniminoxiden (WO 02/33025 A2).

Die EP 0 737 659 A1, EP 0 727 660 A1, US 2012/0101295 A1, US 2009/0114878 A1 nennen weitere Anwendungsmöglichkeiten verschiedener Klassen von Chinonmethiden

Während es durch den Einsatz der Chinonmethide möglich ist, die Toxizität anderer Inhibitoren zu umgehen, ist ihre Herstellung (beschrieben etwa in der EP 2 243 764 A1) recht aufwendig.

Aufgabe der vorliegenden Erfindung war es deshalb, eine neue Klasse von Polymerisationsinhibitoren zur Verfügung zu stellen. Auch sollten diese einfacher zugänglich sein als die Chinonmethide.

Es wurde nun überraschend festgestellt, dass bestimmte Verbindungen, nämlich jene der nachfolgend definierten chemischen Struktur (I), die hier auch als "phenolische (Thio)Acetale" bezeichnet werden, diese Aufgabe lösen. Diese neue Klasse von Inhibitoren zeichnet sich durch eine höhere Temperaturbeständigkeit aus und ist außerdem leichter zugänglich als etwa Chinonmethide.

Ähnliche Inhibitoren werden auch im Stand der Technik diskutiert. So beschreiben Skurko *et al*. [M.R. Skurko, G.V. Leplyanin, S.S. Zlotskiy, P.S. Belov, D.L. Rakhmankulov, S.R. Rafikov, Plasticheskie Massy 1979, 6, 7 - 9] den Einfluss von phenolischen Cycloacetalen auf die Polymerisierung von Styrol, Vinylacetat oder Methylmethacrylat. Es wird exemplarisch die Inhibitoraktivität dieser cycloacetalischen Phenole bei der Photopolymerisation von Methylmethacrylat beschrieben und mit der inhibitorischen Aktivität von 2,6-Di-*tert*-Butyl-*p*-kresol ("BHT") verglichen.

Die phenolischen (Thio)Acetale der vorliegenden Erfindung unterscheiden sich dadurch in ihrer Struktur von denen, welche Skurko *et al.* beschreiben, dass die Acetalfunktion der erfindungsgemäßen Verbindungen der chemischen Struktur **(I)** nicht cyclisch ist.

Es wurde überraschend festgestellt, dass die erfindungsgemäßen Verbindungen der chemischen Struktur **(I)** eine deutlich verbesserte inhibitorische Aktivität gegenüber BHT und den von Skurko *et al.* beschriebenen phenolischen Cycloacetalen aufweisen.

### Detaillierte Beschreibung der Erfindung

Die vorliegende Erfindung betrifft in einem ersten Aspekt ein Verfahren zur Inhibierung der Polymerisation von **(B),** dadurch gekennzeichnet, dass **(A)** mit **(B)** vermischt und dadurch eine Zusammensetzung **(AB)** erhalten wird, wobei
**(A)** mindestens eine Verbindung der chemischen Struktur **(I)** ist mit 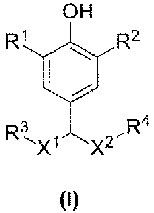
   wobei X¹ und X² unabhängig voneinander jeweils S oder O ist; und bevorzugt gilt X¹ = X² = O oder X¹ = X² = S; noch bevorzugter gilt X¹ = X² = O;
   wobei R¹, R² unabhängig voneinander jeweils ausgewählt aus der Gruppe bestehend aus Wasserstoff, Alkylgruppe mit 1 bis 18 Kohlenstoffatomen, Cycloalkylgruppe mit 3 bis 15 Kohlenstoffatomen, Arylgruppe mit 6 bis 14 Kohlenstoffatomen sind;
   wobei R³, R⁴ unabhängig voneinander jeweils ausgewählt aus der Gruppe bestehend aus
      -(CH₂)ₐ[O(CH₂)_{b}]_{c}R⁵, wobei R⁵ = H oder OH; a, c unabhängig voneinander jeweils eine ganze Zahl aus dem Bereich 1 bis 10 ist; und b = 1, 2, 3 oder 4 ist;
      Alkylgruppe mit 1 bis 18 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR⁶, -OR⁷, -SR⁸, -NR⁹R¹⁰, -CN, Arylgruppe mit 6 bis 14 Kohlenstoffatomen substituiert sein kann;
      Cycloalkylgruppe mit 3 bis 15 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹¹, -OR¹², -SR¹³, -NR¹⁴R¹⁵, -CN substituiert sein kann;
      Arylgruppe mit 6 bis 14 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹⁶, -OR¹⁷, -SR¹⁸, -NR¹⁹R²⁰, -CN, Alkylgruppe mit 1 bis 6 Kohlenstoffatomen substituiert sein kann;
      Heteroarylgruppe mit 1 bis 10 Kohlenstoffatomen, bei welcher mindestens ein an ein Kohlenstoffatom gebundener Wasserstoffrest durch einen Rest ausgewählt aus der Gruppe bestehend
         aus -F, -Cl, -Br, -I, -NH, -OH, -SH, -NHR²¹, -OR²², -SR²³, -NR²⁴R²⁵, -CN, Alkylgruppe mit 1 bis 6 Kohlenstoffatomen substituiert sein kann;
      Heterocycloalkylgruppe mit 2 bis 14 Kohlenstoffatomen, bei welcher mindestens ein an ein Kohlenstoffatom gebundener Wasserstoffrest durch einen Rest ausgewählt aus der Gruppe bestehend aus -F, -Cl, -Br, -I, -NH, -OH, -SH, -NHR²⁶, -OR²⁷, -SR²⁸, ⁻NR²⁹R³⁰, -CN substituiert sein kann;
         wobei R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ unabhängig voneinander jeweils aus der Gruppe bestehend aus Alkylgruppe mit 1 bis 18 Kohlenstoffatomen, Cycloalkylgruppe mit 3 bis 15 Kohlenstoffatomen ausgewählt sind;
      sind;
      und wobei
**(B)** mindestens ein olefinisch ungesättigtes Monomer ist,
   wobei **(A)** in einer solchen Menge eingesetzt wird, dass das Gesamtgewicht aller im Verfahren eingesetzten Verbindungen der Struktur **(I)** im Bereich von 1 ppm (m/m) bis 100000 ppm (m/m) liegt, jeweils bezogen auf das Gesamtgewicht aller im Verfahren eingesetzten olefinisch ungesättigten Monomere,
   und wobei das olefinisch ungesättigte Monomer ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid ist.

Ein olefinisch ungesättigtes Monomer ist erfindungsgemäß ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; bevorzugt ausgewählt aus der Gruppe bestehend aus Styrol, Divinylbenzol, Vinylacetat, Acrylat, Methacrylat, Acrylsäure, Methacrylsäure, Cyclopentadien; noch bevorzugter ausgewählt aus der Gruppe bestehend aus Styrol, Divinylbenzol, Vinylacetat, Acrylat, Methacrylat, Acrylsäure, Methacrylsäure; sogar noch bevorzugter ausgewählt aus der Gruppe bestehend aus Styrol, Divinylbenzol. Am bevorzugtesten ist das olefinisch ungesättigte Monomer Styrol.

Die erfindungsgemäßen Verbindungen der Struktur **(I)** lassen sich nach dem Fachmann bekannten Verfahren synthetisieren (beschrieben etwa in der EP 2 243 764 A1).

Eine "Alkylgruppe" kann im Sinn der Erfindung unverzweigt oder verzweigt sein.

Eine "Alkylgruppe mit 1 bis 18 Kohlenstoffatomen" ist im Sinne der Erfindung insbesondere ausgewählt aus der Gruppe bestehend aus Methyl, Ethyl, *n*-Propyl, *iso*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl.

Eine "Alkylgruppe mit 1 bis 15 Kohlenstoffatomen" ist im Sinne der Erfindung insbesondere ausgewählt aus der Gruppe bestehend aus Methyl, Ethyl, *n*-Propyl, *iso*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl.

Eine "Alkylgruppe mit 1 bis 12 Kohlenstoffatomen" ist im Sinne der Erfindung insbesondere ausgewählt aus der Gruppe bestehend aus Methyl, Ethyl, *n*-Propyl, *iso*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl.

Eine "Alkylgruppe mit 1 bis 6 Kohlenstoffatomen" ist im Sinne der Erfindung insbesondere ausgewählt aus der Gruppe bestehend aus Methyl, Ethyl, *n*-Propyl, *iso*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl.

Der Begriff "Cycloalkylgruppe mit 3 bis 15 Kohlenstoffatomen" bezeichnet im Sinne der Erfindung einen Kohlenwasserstoffrest mit 3 bis 15 gesättigten Kohlenstoffatomen, in welchem mindestens 3 Kohlenstoffatome einen Ring bilden, und welcher neben Wasserstoff und Kohlenstoff keine weiteren Elemente aufweist. Die "Cycloalkylgruppe mit 3 bis 15 Kohlenstoffatomen" ist dabei erfindungsgemäß über ein Kohlenstoffatom, welches Teil des Rings ist oder mit einem Kohlenstoffatom, welches nicht Teil des Ringes ist, mit dem übrigen Molekül verknüpft. Eine "Cycloalkylgruppe mit 3 bis 15 Kohlenstoffatomen" ist im Sinne der Erfindung insbesondere ausgewählt aus der Gruppe bestehend aus Cyclopropyl, Cyclobutyl, Cyclopropylmethyl, Cyclopentyl, Cyclobutylmethyl, Cyclohexyl, Cyclopentylmethyl, Cycloheptyl, Cyclohexylmethyl, Cyclooctyl, 1-Cyclohexylethyl, 2-Cyclohexylethyl, Cyclononyl, Cyclodecyl, Cycloundecyl, Cyclododecyl, Cyclotridecyl, Cyclotetradecyl, Cyclopentadecyl; bevorzugt ist sie ausgewählt aus der Gruppe bestehend aus Cyclopropyl, Cyclobutyl, Cyclopentyl, Cyclohexyl, Cycloheptyl, Cyclooctyl.

Eine "Arylgruppe mit 6 bis 14 Kohlenstoffatomen" weist im Sinne der Erfindung einen oder mehrere aromatische Kerne auf und ist im Sinne der Erfindung insbesondere ausgewählt aus der Gruppe bestehend aus Phenyl, 1-Naphthyl, 2-Naphthyl, 9-Anthryl, 9-Phenanthryl. Bevorzugt ist eine Arylgruppe mit 6 bis 14 Kohlenstoffatomen eine Phenylgruppe.

Der Begriff "Heteroarylgruppe mit 1 bis 10 Kohlenstoffatomen" bezeichnet im Sinne der Erfindung insbesondere einen aromatischen Rest, welcher innerhalb des aromatischen Systems mindestens ein Heteroatom ausgewählt aus der Gruppe bestehend aus O, N, S, Se aufweist. Die "Heteroarylgruppe mit 1 bis 10 Kohlenstoffatomen" ist dabei dabei über ein Heteroatom, welches Teil des aromatischen Systems ist oder über ein Kohlenstoffatom, welches Teil des aromatischen Systems ist, mit dem übrigen Molekül verknüpft. Die "Heteroarylgruppe mit 1 bis 10 Kohlenstoffatomen" ist im Sinne der Erfindung insbesondere ausgewählt aus der Gruppe bestehend aus 2-Pyridyl, 3-Pyridyl, 4-Pyridyl, 2-Thienyl, 3-Thienyl, 2-Pyrryl, 3-Pyrryl, 2-Furyl, 3-Furyl, 1-Imidazolyl, 2-Imidazolyl, 3-Imidazolyl, 1-Triazolyl, 2-Triazolyl, 3-Triazolyl, 1-Thiazolyl, 2-Thiazolyl, 3-Thiazolyl, 4-Thiazolyl, 5-Thiazolyl, 1-Tetrazolyl, 2-Tetrazolyl, 3-Tetrazolyl.

Der Begriff "Heterocycloalkylgruppe mit 2 bis 14 Kohlenstoffatomen" bezeichnet im Sinne der Erfindung eine Cycloalkylgruppe mit 3 bis 15 Kohlenstoffatomen, bei welcher mindestens eine im cyclischen System befindliche Methylengruppe (also eine "-CH₂-"-Funktion) durch eine Funktion ausgewählt aus der Gruppe bestehend aus -O-, -NH-, -S-, -Se- ersetzt wurde. Die "Heterocycloalkylgruppe mit 2 bis 14 Kohlenstoffatomen" ist dabei über ein gesättigtes Kohlenstoffatom mit dem übrigen Molekül verknüpft. Alternativ kann sich erfindungsgemäß die "Heterocycloalkylgruppe mit 2 bis 14 Kohlenstoffatomen" auch dadurch ergeben, dass in der Cycloalkylgruppe mit 3 bis 15 Kohlenstoffatomen eine im cyclischen System befindliche Methylengruppe durch eine Funktion -(N-R*)- ersetzt wurde, wobei R* das übrige Molekül darstellt. Im letzten Fall findet die Verknüpfung zum übrigen Molekül also über das Heteroatom Stickstoff statt.

Die "Heterocycloalkylgruppe mit 2 bis 14 Kohlenstoffatomen" ist im Sinne der Erfindung insbesondere ausgewählt aus Aziridinyl, Oxiranyl, Thiiranyl,1-Azetidinyl, 2-Azetidinyl, 3-Azetidinyl, 2-Oxetanyl, 3-Oxetanyl, 2-Thietanyl, 3-Thietanyl, 1-Azolidinyl, 2-Azolidinyl, 3-Azolidinyl, 2-Oxolanyl, 3-Oxolanyl, 2-Thiolanyl, 3-Thiolanyl, 1-Pyrrolidinyl, 2-Pyrrolidinyl, 3-Pyrrolidinyl, 2-Tetrahydrofuryl, 3-Tetrahydrofuryl, 2-Tetrahydrothiohenyl, 3-Tetrahydrothiophenyl, 2-Morpholinyl, 3-Morpholinyl, 4-Morpholinyl,1-Piperidyl, 2-Piperidyl, 3-Piperidyl, 4-Piperidyl, 2-Tetrahydropyryl, 3-Tetrahydropyryl, 4-Tetrahydropyryl, 2-Tetrahydrothiopyryl, 3-Tetrahydrothiopyryl, 4-Tetrahydrothiopyryl, 1-Piperazyl, 2-Piperazyl.

Der Begriff "phenolisches (Thio)Acetal" bezeichnet erfindungsgemäß phenolische Acetale, phenolische Thioacetale und phenolische gemischte Acetale. Phenolische Acetale zeichnen sich dadurch aus, dass in der Verbindung der chemischen Struktur **(I)** X¹ = X² = O ist. Phenolische Thioacetale zeichnen sich dadurch aus, dass in der Verbindung der chemischen Struktur **(I)** X¹ = X² = S ist. Phenolische gemischte Acetale zeichnen sich dadurch aus, dass einer der Reste X¹ und X² = O ist und der andere S ist. Bevorzugt handelt es sich erfindungsgemäß bei den phenolischen (Thio)Acetalen um phenolische Acetale, das heißt um Verbindungen der chemischen Struktur **(I),** bei denen X¹ = X² = O ist.

In einer bevorzugten Ausführungsform des Verfahrens gemäß des ersten Aspekt der Erfindung sind X¹ = X² = O; und R¹, R² sind unabhängig voneinander jeweils ausgewählt aus der Gruppe bestehend aus Wasserstoff, Alkylgruppe mit 1 bis 18 Kohlenstoffatomen; und R³, R⁴ sind unabhängig voneinander jeweils ausgewählt aus der Gruppe bestehend aus Alkylgruppe mit 1 bis 18 Kohlenstoffatomen, Arylgruppe mit 6 bis 14 Kohlenstoffatomen, Benzyl. Noch bevorzugter sind im Verfahren gemäß des ersten Aspekt der Erfindung X¹ = X² = O; R¹ und R² gleich und jeweils ausgewählt aus der Gruppe bestehend aus Methyl, *n*-Propyl, *iso*-Propyl, *tert*-Butyl sind und gleichzeitig sind auch R³ und R⁴ gleich und jeweils eine Alkylgruppe mit 1 bis 6 Kohlenstoffatomen oder jeweils Phenyl oder jeweils Benzyl.

Am bevorzugtesten ist im Verfahren gemäß des ersten Aspekt der Erfindung X¹ = X² = O; R¹ = *tert*-Butyl; R² = *tert*-Butyl; und R³ und R⁴ sind gleich und jeweils ausgewählt aus der Gruppe bestehend aus Methyl, Ethyl, *n*-Propyl, *iso*-Propyl, *n*-Butyl, *n*-Pentyl, *n*-Hexyl, Phenyl, Benzyl, wobei Methyl, Ethyl, *n*-Propyl besonders hervorzuheben sind.

In einer ersten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = Methyl, wobei die Verbindung der Struktur **(I)** dann die Struktur **(I-1)** aufweist: 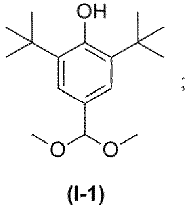 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer zweiten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = Ethyl, wobei die Verbindung der Struktur **(I)** dann die Struktur **(I-2)** aufweist: 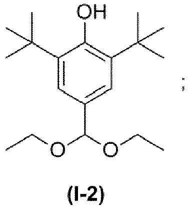 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer dritten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = *n*-Propyl, wobei die Verbindung der Struktur **(I)** dann die Struktur **(I-3)** aufweist: 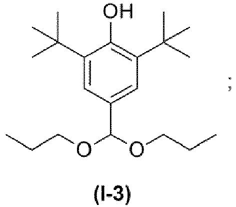 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer vierten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = *iso*-Propyl, wobei die Verbindung der Struktur **(I)** dann die Struktur **(I-4)** aufweist: 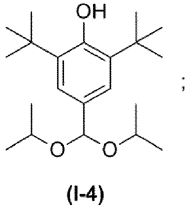 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer fünften ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = *n*-Butyl, wobei die Verbindung der Struktur **(I)** dann die Struktur **(I-5)** aufweist: 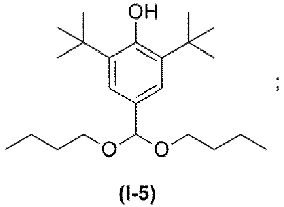 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer sechsten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = *n*-Pentyl, wobei die Verbindung der Struktur **(I)** dann die Struktur **(I-6)** aufweist: 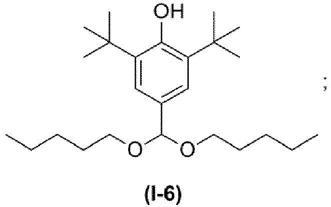 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer siebten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = *n*-Hexyl, wobei die Verbindung der Struktur **(I)** dann die Struktur **(I-7)** aufweist: 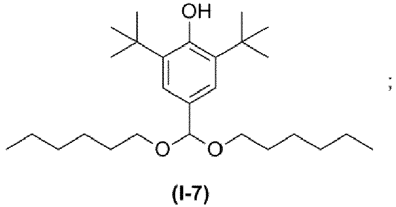 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer achten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = Phenyl, wobei die Verbindung der Struktur **(I)** dann die Struktur **(I-8)** aufweist: 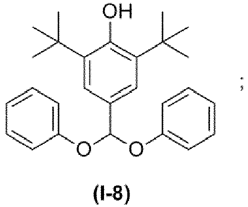 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer neunten ganz besonders bevorzugten Ausführungsform des Verfahrens gemäß des ersten Aspekts der Erfindung ist in der Verbindung der Struktur **(I)** X¹ = X² = O, R¹ und R² = *tert*-Butyl und R³ = R⁴ = Benzyl, wobei die Verbindung der Struktur **(I)** dann die Struktur **(I-9)** aufweist: 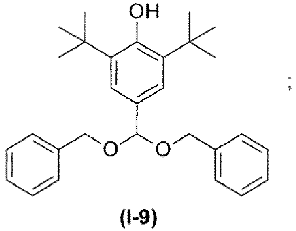 und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

Neben der Tatsache, dass die Verbindungen der chemischen Struktur **(I)** eine deutlich verbesserte inhibitorische Aktivität gegenüber den von Skurko *et al.* beschriebenen Polymerisationsinhibitoren aufweisen, bieten sie einen weiteren überraschenden Vorteil vor allem gegenüber den Chinonmethiden. Die erfindungsgemäßen Verbindungen können nämlich sehr einfach, wie in der EP 2 243 764 A1 beschrieben, in einer Stufe aus dem entsprechenden Aldehyd hergestellt werden. Demgegenüber erfordert die Herstellung der Chinonmethide einen zweiten, energieintensiven Schritt, in welchem der (Thio)Alkohol eliminiert wird.

Die Verbindungen der chemischen Struktur **(I)** zeigen eine sehr gute Polymerisationsinhibierung von olefinisch ungesättigten Monomeren. Dieser Vorteil wirkt sich auch bei höheren Temperaturen aus (≥ 100 °C).

Im Vergleich zur Herstellung der Chinonmethide sind die die Verbindungen der chemischen Struktur **(I)** somit mit einem viel geringeren Aufwand erhältlich, was sie gerade für die großtechnische Anwendung prädestiniert.

Die im erfindungsgemäßen Verfahren vorliegende Temperatur ist somit zwar grundsätzlich nicht beschränkt. Da die Verbindungen der chemischen Struktur **(I)** aber bei hohen Temperaturen angewendet werden können, wird das erfindungsgemäße Verfahren vorteilhafterweise bei einer Temperatur von ≥ 100 °C, bevorzugt 100 bis 160 °C, noch bevorzugter 110 bis 150 °C, noch bevorzugter bei 120 °C durchgeführt.

Der im erfindungsgemäßen Verfahren vorliegende Druck ist grundsätzlich nicht beschränkt, liegt vorteilhafterweise aber im Bereich von 0.01 - 10 bar, bevorzugter 0.1 - 2 bar.

Im erfindungsgemäßen Verfahren kann **(A)** gasförmig, als Feststoff (z. B. als Pulver) oder als Flüssigkeit, insbesondere als Feststoff (z. B. als Pulver) oder als Flüssigkeit, bevorzugt als Flüssigkeit eingesetzt werden. Wird **(A)** im erfindungsgemäßen Verfahren als Flüssigkeit eingesetzt, dann wird **(A)** insbesondere als Schmelze oder in **(C)** mindestens einem Lösungsmittel eingesetzt, wobei der Einsatz in **(C)** mindestens einem Lösungsmittel besonders bevorzugt ist.

Als Lösungsmittel im erfindungsgemäßen Verfahren eignen sich alle Stoffe, in denen **(A)** im gewünschten Konzentrationsbereich löslich ist, und die sowohl mit **(A)** verträglich sind als auch keinen störenden Einfluss auf das erfindungsgemäße Verfahren haben, insbesondere unpolare, bevorzugt unpolare aromatische oder aliphatische Lösungsmittel.

Bevorzugt ist das Lösungsmittel ausgewählt aus der Gruppe bestehend aus Benzol, einfach oder mehrfach alkylierten Aromaten, Alkanen mit einer Kohlenstoffzahl von 6 bis 15, Cycloalkanen mit einer Kohlenstoffzahl von 6 bis 15, hochsiedenden Kohlenwasserstoffschnitten, Ethern mit einer Kohlenstoffzahl von 6 bis 15, Estern mit einer Kohlenstoffzahl von 6 bis 15.

Noch bevorzugter ist das Lösungsmittel im erfindungsgemäßen Verfahren ausgewählt aus der Gruppe bestehend aus Benzol, Toluol, Ethylbenzol, Xylol, Styrol, hochsiedende aromatische Kohlenwasserstoffschnitte. Besonders bevorzugt ist das Lösungsmittel im erfindungsgemäßen Verfahren ausgewählt aus der Gruppe bestehend aus Toluol, Ethylbenzol, Xylol und Styrol. Es kann in einer alternativen Ausführungsform des erfindungsgemäßen Verfahrens sogar das olefinisch ungesättigte Monomer selbst als Lösungsmittel dienen.

Wird im erfindungsgemäßen Verfahren **(A)** gelöst in **(C)** eingesetzt, so liegt bevorzugt in der resultierenden Lösung **(AC)** das Verhältnis (m/m) des Gesamtgewichts aller Verbindungen der Struktur **(I)** in der Lösung **(AC)** zum Gesamtgewicht aller Lösungsmittel in der Lösung **(AC)** im Bereich von 1 : 1000 bis 100 : 1, bevorzugter im Bereich von 1 : 100 bis 10 : 1, noch bevorzugter im Bereich von 1 : 10 bis 3 : 1.

**(B)** wird im erfindungsgemäßen Verfahren gasförmig, als Flüssigkeit oder als Feststoff, insbesondere gasförmig oder als Flüssigkeit, bevorzugt als Flüssigkeit eingesetzt. Wird **(B)** als Flüssigkeit eingesetzt, ist es noch bevorzugter, dass es als Schmelze oder in Lösung eingesetzt wird.

Besonders bevorzugt wird **(B)** in einer Lösung eingesetzt. Eine solche Lösung ist in einer ersten ganz besonders bevorzugten Ausführungsform ein Prozessstrom, wie er bei Crackingprozessen erhalten wird. In einem solchen Prozessstrom liegen dann alle olefinisch ungesättigten Monomere insbesondere mit einem Anteil im Bereich von 0.0001 Gew.-% bis 15 Gew.-%, bezogen auf das Gesamtgewicht des Prozesstroms, vor. In einer alternativen zweiten ganz besonders bevorzugten Ausführungsform kann die Lösung ein Prozessstrom sein, wie er bei der Herstellung des olefinisch ungesättigten Monomeren selbst anfällt. In einem solchen Prozessstrom liegen dann alle olefinisch ungesättigten Monomere insbesondere mit einem Anteil zwischen 15 und 100 Gew.-% vor, bevorzugt zwischen 70 und 99.9 Gew-%, noch bevorzugter zwischen 70 und 99 Gew.-%, jeweils bezogen auf das Gesamtgewicht des Prozesstroms, vor.

Im Sinne der Erfindung kann die Vermischung von **(A)** mit **(B)** in jeder dem Fachmann bekannten Form erfolgen. Insbesondere bedeutet **"(A)** wird mit **(B)** vermischt", dass **(A)** zu **(B)** gegeben oder **(B)** zu **(A)** gegeben wird.

Vorteilhafterweise erfolgt die Zugabe von **(A)** im erfindungsgemäßen Verfahren in jeglichen Zulaufstrom (feed-stream) oder Ableitung einer Destillationskolonne, in die Zu- und Ableitung eines Wärmetauschers oder in die Zu- und Ableitung eines Verdampfers ("Aufkochers") oder in die Zu- und Ableitung eines Kondensators oder in die Zu- und Ableitung eines Reaktors. Darüber hinaus kann im erfindungsgemäßen Verfahren **(A)** auch zu Lagertanks, die einen **(B)** enthaltenden Prozessstrom enthalten, gegeben werden. Die Vermischung von **(A)** und **(B)** kann sowohl vor als auch während eines Verfahrens, wie beispielsweise Herstellungs- oder Reinigungsverfahrens, erfolgen.

Im erfindungsgemäßen Verfahren wird eine effektive Menge von **(A)** eingesetzt. Unter dem Begriff "effektive Menge von **(A)"** wird im Rahmen dieser Erfindung die Menge an **(A)** verstanden, die notwendig ist, um die unerwünschte Polymerisation von **(B)** zu verzögern bzw. zu verhindern. Diese effektive Menge ist abhängig von den Bedingungen, unter denen **(B)** gelagert oder gehandhabt wird und kann leicht vom Fachmann von Fall zu Fall bestimmt werden. Beispielsweise ist beim Cracken auf Grund der höheren Temperaturen eine höhere Menge an **(A)** notwendig als bei der Lagerung von **(B)** z. B. bei Raumtemperatur.

Im erfindungsgemäßen Verfahren wird **(A)** in einer solchen Menge eingesetzt, dass das Gesamtgewicht aller im erfindungsgemäßen Verfahren eingesetzten Verbindungen der Struktur **(I)** im Bereich von 1 ppm (m/m) bis 100000 ppm (m/m), bevorzugt im Bereich von 1 ppm (m/m) bis 50000 ppm (m/m), bevorzugter im Bereich von 10 ppm bis 10000 ppm (m/m), noch bevorzugter im Bereich von 100 ppm bis 5000 ppm (m/m), und am bevorzugtesten im Bereich von 200 ppm bis 500 ppm (m/m) liegt, jeweils bezogen auf das Gesamtgewicht aller im Verfahren eingesetzten olefinisch ungesättigten Monomere.

Im erfindungsgemäßen Verfahren kann **(B)** zusätzlich mit einer weiteren Verbindung **(D)** vermischt werden. **(D)** ist dabei mindestens eine Verbindung ausgewählt aus der Gruppe bestehend aus Hydroxylaminen, Oximen, sterisch gehinderten *N*-Oxyl-Verbindungen, Chinonmethiden, Nitroaromaten, Nitrosoaromaten, Benzochinonen, Hydrochinonen, Phenothiazinen, phenolischen Antioxidantien, *N*,*N'*-Dialkyl-*p*-phenylendiaminen, *N*,*N*'-Dialkyl-*p*-benzochinondiimiden.

**(D)** ist dabei insbesondere mindestens eine Verbindung ausgewählt aus der Gruppe bestehend aus Hydroxylaminen, sterisch gehinderten *N*-Oxyl-Verbindungen, Chinonmethiden, Nitroaromaten, Nitrosoaromaten, Benzochinonen, Hydrochinonen, Phenothiazinen, phenolischen Antioxidantien, *N*,*N*'-Dialkyl-*p*-phenylendiaminen, *N*,*N*'-Dialkyl-*p*-benzochinondiimiden.

**(D)** ist dabei bevorzugt mindestens eine Verbindung ausgewählt aus der Gruppe bestehend aus sterisch gehinderten *N*-Oxyl-Verbindungen, Chinonmethiden, Nitroaromaten.

Die Verbindung **(D)** stellt dabei einen weiteren Polymerisationsinhibitor dar und kann die Inhibitionswirkung weiter verbessern. Das zusätzliche Mischen von **(B)** mit **(D)** ist dabei nicht besonders beschränkt und etwa gemäß einer der nachfolgenden Möglichkeiten (i), (ii), (iii) durchführbar.
(i) So kann in einer Ausführungsform erst **(D)** mit **(A)** vermischt werden, und die resultierende Mischung **(DA)** wird dann mit **(B)** vermischt.
(ii) In einer anderen Ausführungsform wird erst **(D)** mit **(B)** vermischt, und die resultierende Mischung **(DB)** wird dann mit **(A)** vermischt.
(iii) In einer weiteren Ausführungsform wird erst **(A)** mit **(B)** vermischt, und die resultierende Mischung **(AB)** wird dann mit **(D)** vermischt.

Die Ausführungsform (i) ist besonders bevorzugt.

Hydroxylamine sind zum Beispiel in der US 5,282,957 beschrieben. Erfindungsgemäß besonders bevorzugte Hydroxylamine weisen die folgenden chemischen Strukturen **(II-1)** oder **(II-2)** auf: 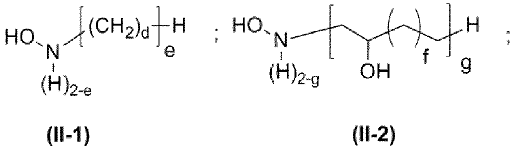
wobei e, g unabhängig voneinander jeweils 1 oder 2 ist;
wobei d, f unabhängig voneinander jeweils 0, 1, 2, 3, 4, 5, 6, 7, 8, 9 oder 10 ist; und
bevorzugt d, f unabhängig voneinander jeweils 1, 2, 3, 4, 5, 6, 7, 8, 9 oder 10 ist;
und wobei Diethylhydroxylamin ein ganz besonders bevorzugtes Hydroxylamin ist.

Chinonmethide werden zum Beispiel in folgenden Schriften beschrieben: US 4,003,800, US 4,040,911, EP 0 737 659, EP 0 737 660, WO 99/48896, US 2005/0027150, EP 2 055 691 A1.

Eine erste Gruppe von erfindungsgemäß bevorzugten Chinonmethiden sind die Verbindungen gemäß der folgenden chemischen Struktur **(III)** mit: 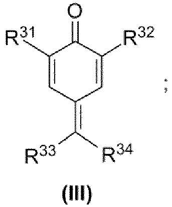
wobei R³¹, R³² unabhängig voneinander jeweils ausgewählt aus der Gruppe bestehend aus Wasserstoff, Alkylgruppe mit 1 bis 15 Kohlenstoffatomen sind;
wobei R³³, R³⁴ unabhängig voneinander jeweils ausgewählt aus der Gruppe bestehend aus Wasserstoff, -CN, -COOR³⁵, -COR³⁶, -OCOR³⁷, -CONR³⁸R³⁹, -PO(OR⁴⁰)₂, Alkylgruppe mit 1 bis 15 Kohlenstoffatomen, Cycloalkylgruppe mit 3 bis 15 Kohlenstoffatomen, Arylgruppe mit 6 bis 14 Kohlenstoffatomen, Heteroarylgruppe mit 1 bis 10 Kohlenstoffatomen, wobei die Arylgruppe mit 6 bis 14 Kohlenstoffatomen ihrerseits substituiert sein kann mit mindestens einer Alkylgruppe mit 1 bis 6 Kohlenstoffatomen, sind;
   wobei R³⁵, R³⁶, R³⁷, R³⁸, R³⁹, R⁴⁰ unabhängig voneinander jeweils ausgewählt aus der Gruppe bestehend aus Wasserstoff, Alkylgruppe mit 1 bis 15 Kohlenstoffatomen, Cycloalkylgruppe mit 3 bis 15 Kohlenstoffatomen, Arylgruppe mit 6 bis 14 Kohlenstoffatomen sind.

Eine besonders bevorzugte Verbindung der chemischen Struktur **(III)** ist dabei jene, für die gilt: R³¹ = R³² = *tert*-Butyl, R³³ = H und R³⁴ = Phenyl.

Eine zweite Gruppe von erfindungsgemäß bevorzugten Chinonmethiden sind die Verbindungen gemäß der folgenden chemischen Struktur **(IV)** mit: 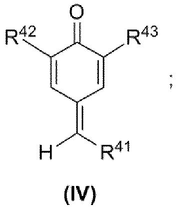 wobei
R⁴¹ ausgewählt aus der Gruppe bestehend aus -F, -Cl, -Br, -I, -O-R⁴⁴, -S-R⁴⁵ ist;
R⁴², R⁴³, R⁴⁴, R⁴⁵ unabhängig voneinander jeweils ausgewählt aus der Gruppe bestehend aus Wasserstoff, Alkylgruppe mit 1 bis 15 Kohlenstoffatomen, Cycloalkylgruppe mit 3 bis 15 Kohlenstoffatomen, Arylgruppe mit 6 bis 14 Kohlenstoffatomen sind.

Eine besonders bevorzugte Verbindung der chemischen Struktur **(IV)** ist dabei jene für R⁴¹ = -O-R⁴⁴, wobei R⁴⁴ eine Alkylgruppe mit 1 bis 6 Kohlenstoffatomen, noch bevorzugter Methyl, *n*-Propyl oder *iso*-Propyl ist; und wobei R⁴², R⁴³ unabhängig voneinander jeweils ausgewählt aus der Gruppe bestehend Methyl, *tert*-Butyl, noch bevorzugter beide jeweils *tert*-Butyl sind.

Sterisch gehinderten *N*-Oxyl-Verbindungen sind beispielsweise in EP 2 055 691 A1 und US 5,254,760 beschrieben.

Erfindungsgemäß bevorzugte sterisch gehinderte *N*-Oxyl-Verbindungen sind jene der chemischen Strukturen **(V), (VI), (VII), (VIII)** mit: 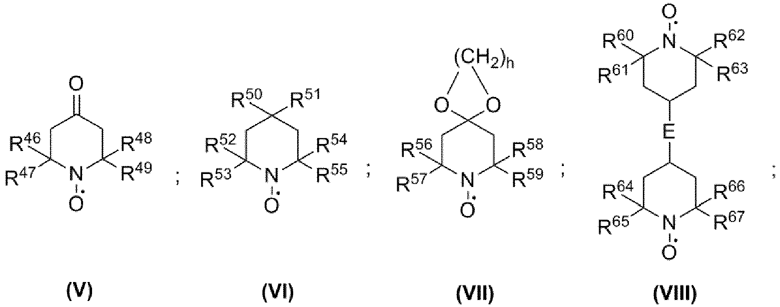 wobei
R⁴⁶, R⁴⁷, R⁴⁸, R⁴⁹, R⁵², R⁵³, R⁵⁴, R⁵⁵, R⁵⁶, R⁵⁷, R⁵⁸, R⁵⁹, R⁶⁰, R⁶¹, R⁶², R⁶³, R⁶⁴, R⁶⁵, R⁶⁶, R⁶⁷ unabhängig voneinander jeweils ausgewählt sind aus der Gruppe bestehend aus Methyl, Ethyl, *n*-Propyl, *iso*-Propyl, *n*-Butyl, *iso*-Butyl, sec-Butyl, *tert*-Butyl und besonders bevorzugt alle jeweils Methyl sind;
R⁵⁰, R⁵¹ unabhängig voneinander jeweils ausgewählt sind aus der Gruppe bestehend aus Wasserstoff, Alkylgruppe mit 1 bis 18 Kohlenstoffatomen, -NR⁶⁸R⁶⁹, -F, -Cl, -Br, -I, -OR⁷⁰, -COOR⁷¹, -O-CO-NHR⁷², -NH-CO-R⁷³, -O-CO-R⁷⁴,
   wobei R⁶⁸, R⁶⁹, R⁷⁰, R⁷¹, R⁷², R⁷³, R⁷⁴ unabhängig voneinander jeweils ausgewählt aus der Gruppe bestehend aus Wasserstoff, Alkylgruppe mit 1 bis 18 Kohlenstoffatomen, Arylgruppe mit 6 bis 14 Kohlenstoffatomen, Heteroarylgruppe mit 1 bis 10 Kohlenstoffatomen, Benzyl sind, wobei die Arylgruppe mit 6 bis 14 Kohlenstoffatomen und die Benzylgruppe ihrerseits substituiert sein können mit mindestens einer Alkylgruppe mit 1 bis 6 Kohlenstoffatomen;
und wobei h = 1, 2, 3, 4, 5, oder 6 ist;
und wobei E ein zweiwertiger Rest ausgewählt aus der Gruppe bestehend aus Alkylengruppe mit 1 bis 18 Kohlenstoffatomen, bei welcher mindestens ein Wasserstoffrest durch eine Arylgruppe mit 6 bis 14 Kohlenstoffatomen substituiert sein und welche außerdem auch mindestens ein Heteroatom ausgewählt aus O, S, N aufweisen kann, Arylengruppe mit 6 bis 14 Kohlenstoffatomen, bei welcher mindestens ein Wasserstoffrest durch eine Alkylgruppe mit 1 bis 6 Kohlenstoffatomen substituiert sein und welche außerdem auch mindestens ein Heteroatom ausgewählt aus O, S, N aufweisen kann, ist.

Außerdem kann die sterisch gehinderte *N*-Oxyl-Verbindung erfindungsgemäß auch ausgewählt werden aus der Gruppe bestehend aus *N*-(1-Oxyl-2,2,6,6-tetramethylpiperidin-4-yl)-caprolactam, *N*-(1-Oxyl-2,2,6,6-tetramethylpiperidin-4-yl)-dodecylsuccinimid, 2,4,6-Tris-[*N*-butyl-*N*-(1-oxyl-2,2,6,6-tetramethylpiperidin-4-yl]-*S*-triazin, 4,4'-Ethylenbis(1-oxyl-2,2,6,6-tetramethylpiperazin-3-on).

Die erfindungsgemäß noch bevorzugteren sterisch gehinderte *N*-Oxyl-Verbindungen sind neben *N*-(1-Oxyl-2,2,6,6-tetramethylpiperidin-4-yl)-caprolactam, *N*-(1-Oxyl-2,2,6,6-tetramethylpiperidin-4-yl)-dodecylsuccinimid, 2,4,6-Tris-[*N*-butyl-*N*-(1-oxyl-2,2,6,6-tetramethylpiperidin-4-yl]-*S*-triazin 4,4'-Ethylenbis(1-oxyl-2,2,6,6-tetramethylpiperazin-3-on) jene der vorgenannten chemischen Strukturen **(V), (VI), (VII), (VIII)** wobei
R⁴⁶, R⁴⁷, R⁴⁸, R⁴⁹, R⁵², R⁵³, R⁵⁴, R⁵⁵, R⁵⁶, R⁵⁷, R⁵⁸, R⁵⁹, R⁶⁰, R⁶¹, R⁶², R⁶³, R⁶⁴, R⁶⁵, R⁶⁶, R⁶⁷ alle Methyl sind;
R⁵⁰ = Wasserstoff ist;
R⁵¹ ausgewählt aus der Gruppe bestehend aus Wasserstoff, Alkylgruppe mit 1 bis 18 Kohlenstoffatomen, -NR⁶⁸R⁶⁹, -F, -Cl, -Br, -I, -OR⁷⁰, -COOR⁷¹, -O-CO-NHR⁷², -NH-CO-R⁷³, -O-CO-R⁷⁴,
wobei R⁶⁸, R⁶⁹, R⁷⁰, R⁷¹, R⁷², R⁷³, R⁷⁴ unabhängig voneinander jeweils ausgewählt aus der Gruppe bestehend aus Wasserstoff, Alkylgruppe mit 1 bis 18 Kohlenstoffatomen, Arylgruppe mit 6 bis 14 Kohlenstoffatomen, Heteroarylgruppe mit 1 bis 10 Kohlenstoffatomen, Benzyl sind;
ist;
und wobei E ein zweiwertiger Rest ausgewählt aus der Gruppe bestehend aus Alkylengruppe mit 1 bis 18 Kohlenstoffatomen, bei welcher mindestens ein Wasserstoffrest durch eine Arylgruppe mit 6 bis 14 Kohlenstoffatomen substituiert sein und welche außerdem auch mindestens ein Heteroatom ausgewählt aus O, S, N aufweisen kann,
Arylengruppe mit 6 bis 14 Kohlenstoffatomen, bei welcher mindestens ein Wasserstoffrest durch eine Alkylgruppe mit 1 bis 6 Kohlenstoffatomen substituiert sein und welche außerdem auch mindestens ein Heteroatom ausgewählt aus O, S, N aufweisen kann, ist.

Erfindungsgemäß ganz besonders bevorzugte sterisch gehinderte *N*-Oxyl-Verbindungen sind ausgewählt aus der Gruppe bestehend aus 2,2,6,6-Tetramethylpiperidin-*N*-oxyl ("TEMPO"), 4-Acetamido-2,2,6,6-tetramethylpiperidin-*N*-oxyl ("AA-TEMPO"), 4-Hydroxy-2,2,6,6-tetramethylpiperidin-*N*-oxyl ("4-OH-TEMPO"), 4-Oxo-2,2,6,6-tetramethylpiperidin-*N*-oxyl ("Oxo-TEMPO"), 1-Oxyl-2,2,6,6-tetramethylpiperidin-4-yl-acetat; 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-4-*tert*-butylbenzoat, Bis(1-oxyl-2,2,6,6-tetramethylpiperidin-4-yl)-succinat, Bis(1-oxyl-2,2,6,6-tetramethylpiperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-*n*-butylmalonat, Bis(1-oxyl-2,2,6,6-tetramethylpiperidin-4-yl)-phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethylpiperidin-4-yl)-hexahydroterephthalat, *N*,*N*'-Bis(1-oxyl-2,2,6,6-tetramethylpiperidin-4-yl)-adipamid, *N*-(1-Oxyl-2,2,6,6-tetramethylpiperidin-4-yl)-caprolactam, *N*-(1-Oxyl-2,2,6,6-tetramethylpiperidin-4-yl)-dodecylsuccinimide, 2,4,6-Tris-[*N*-butyl-*N*-(1-oxyl-2,2,6,6-tetramethylpiperidin-4-yl]-S-triazin, 4,4'-Ethylenbis(1-oxyl-2,2,6,6-tetramethylpiperazin-3-one), der Verbindung der vorgenannten chemischen Struktur **(VII),** in welcher R⁵⁶ = R⁵⁷ = R⁵⁸ = R⁵⁹= Methyl ist und h = 2 ist, einer Verbindung der vorgenannten chemischen Struktur **(VI),** in welcher R⁵² = R⁵³ = R⁵⁴ = R⁵⁵ = Methyl, R⁵⁰ = Wasserstoff, R⁵¹ = -OR⁷⁰, wobei R⁷⁰ = Alkylgruppe mit 1 bis 6 Kohlenstoffatomen ist; bevorzugt ist dabei R⁷⁰ eine Alkylgruppe die ausgewählt aus der Gruppe bestehend aus Methyl, Ethyl, *n*-Propyl, *iso*-Propyl, *n*-Butyl, *iso*-Butyl, sec-Butyl, *tert-*Butyl ist, wobei 4-*n*-Butoxy-2,2,6,6-tetramethylpiperidin-*N*-oxyl ("4-*n*-Butoxy-TEMPO") hervorzuheben ist,

Die erfindungsgemäß bevorzugtesten sterisch gehinderten *N*-Oxyl-Verbindungen sind 2,2,6,6-Tetramethylpiperidin-*N*-oxyl ("TEMPO"), 4-Acetamido-2,2,6,6-tetramethylpiperidin-*N*-oxyl ("AA-TEMPO"), 4-Hydroxy-2,2,6,6-tetramethylpiperidin-*N*-oxyl ("4-OH-TEMPO"), 4-*n*-Butoxy-2,2,6,6-tetramethylpiperidin-*N*-oxyl ("4-*n*-Butoxy-TEMPO"), 4-Oxo-2,2,6,6-tetramethylpiperidine-*N*-oxyl ("Oxo-TEMPO").

Erfindungsgemäß lassen sich auch Mischungen mehrerer sterisch gehinderter *N*-Oxyl-Verbindungen einsetzen (beschrieben etwa in CN 102516005 A). Das wäre beispielsweise eine Mischung aus TEMPO mit 4-OH-TEMPO im molaren Verhältnis TEMPO : 4-OH-TEMPO = 9 : 1 oder 8 : 2 oder 7 : 3 oder 6 : 4 oder 1 : 1 oder 4 : 6 oder 3 : 7 oder 2 : 8 oder 1 : 9.
Das wäre beispielsweise eine Mischung aus Oxo-TEMPO mit 4-OH-TEMPO im molaren Verhältnis Oxo-TEMPO : 4-OH-TEMPO = 9 : 1 oder 8 : 2 oder 7 : 3 oder 6 : 4 oder 1 : 1 oder 4 : 6 oder 3 : 7 oder 2 : 8 oder 1 : 9.

Das wäre beispielsweise eine Mischung aus Oxo-TEMPO mit 4-*n*-Butoxy-TEMPO im molaren Verhältnis Oxo-TEMPO : 4-*n*-Butoxy-TEMPO = 9 : 1 oder 8 : 2 oder 7 : 3 oder 6 : 4 oder 1 : 1 oder 4 : 6 oder 3 : 7 oder 2 : 8 oder 1 : 9.

Das wäre beispielsweise eine Mischung aus Oxo-TEMPO mit TEMPO im molaren Verhältnis Oxo-TEMPO : TEMPO = 9 : 1 oder 8 : 2 oder 7 : 3 oder 6 : 4 oder 1 : 1 oder 4 : 6 oder 3 : 7 oder 2 : 8 oder 1 : 9.

Das wäre beispielsweise eine Mischung aus 4-*n*-Butoxy-TEMPO mit 4-OH TEMPO im molaren Verhältnis 4-*n*-Butoxy-TEMPO : 4-OH TEMPO = 9 : 1 oder 8 : 2 oder 7 : 3 oder 6 : 4 oder 1 : 1 oder 4 : 6 oder 3 : 7 oder 2 : 8 oder 1 : 9.

Nitroaromaten sind beispielsweise in der US 5,254,760 beschrieben.

Erfindungsgemäß bevorzugte Nitroaromaten sind ausgewählt aus der Gruppe bestehend aus 1,3-Dinitrobenzol, 1,4-Dinitrobenzol, 2,6-Dinitro-4-methylphenol, 2-Nitro-4-methylphenol, 2,4-Dinitro-1-naphthol, 2,4,6-Trinitrophenol, 2,4-Dinitro-6-methylphenol, 2,4-Dinitrochlorbenzol, 2,4-Dinitrophenol (DNP), 2,4-Dinitro-6-sec-butylphenol (DNBP), 4-Cyano-2-nitrophenol, 3-Jodo-4-cyano-5-nitrophenol, 4,6-Dinitro-*ortho*-kresol (DNOC). Erfindungsgemäß werden die Nitroaromaten dabei noch bevorzugter ausgewählt aus der Gruppe bestehend aus 2,4-Dinitro-6-sec-butylphenol (DNBP), 2,4-Dinitrophenol (DNP), 4,6-Dinitro-*ortho*-cresol (DNOC). Am bevorzugtesten ist der Nitroaromat erfindungsgemäß 2,4-Dinitro-6-sec-butylphenol (DNBP).

Nitrosoaromaten sind beispielsweise in der EP 1 474 377 B1 beschrieben. Nitrosoaromaten werden erfindungsgemäß insbesondere ausgewählt aus der Gruppe bestehend aus Nitrosophenolverbindungen, Nitrosoanilinverbindungen.

Erfindungsgemäß bevorzugte Nitrosophenolverbindungen sind ausgewählt aus der Gruppe bestehend aus 4-Nitrosophenol, 2-Nitrosophenol, 4-Nitroso-*ortho*-kresol, 2-Nitroso-*para*-kresol, 2-*tert*-Butyl-4-nitrosophenol, 4-*tert*-Butyl-2-nitrosophenol, 2,6-Di-*tert*-butyl-4-nitrosophenol, 2-*tert*-Amyl-4-nitrosophenol, 4-*tert*-Amyl-2-nitrosophenol, 2,6-Di-*tert*-amyl-4-nitrosophenol, 2-Nitroso-1-naphthol, 4-Nitroso-1-naphthol, 2-*tert*-Butyl-4-nitroso-1-naphthol, 1-Nitroso-2-naphthol, 6-Nitroso-2-naphthol, 2-*tert*-Amylnitroso-1-naphthol.

Erfindungsgemäß bevorzugte Nitrosoanilinverbindungen sind ausgewählt aus der Gruppe bestehend aus 4-Nitroso-*N*-(1,4-dimethylpentyl)-anilin, 4-Nitroso-*N*-phenylanilin, 4-Nitroso-*N*-*iso*-propylanilin, 4-Nitroso-*N*,*N*-dimethylanilin, 4-Nitroso-*N*,*N*-diethylanilin, 4-Nitroso-*N*,*N*-diphenylanilin, 4-Nitroso-*N*,*N*-di-*iso*-propylanilin, 4-Nitrosoanilin, 2-Nitroso-*N*-(1,4-dimethylpentyl)-anilin, 2-Nitroso-*N*-phenylanilin, 2-Nitroso-*N*,*N*-dimethylanilin, 2-Nitroso-*N*,*N*-diethylanilin, 2-Nitroso-*N*,*N*-diphenylanilin, 2-Nitrosoanilin, 2-*tert*-Butyl-4-nitroso-*N*-(1,4-dimethylpentyl)-anilin, 2-Methyl-4-nitroso-*N*-(1,4-dimethylpentyl)-anilin, 2-*tert*-Butyl-4-nitroso-*N*-phenylanilin, 2-Methyl-4-nitroso-*N*-phenylanilin, 4-*tert*-Butyl-2-nitroso-*N*-(1,4-dimethylpentyl)-anilin, 4-*tert*-Butyl-2-nitroso-*N*-phenylanilin, 4-*tert*-Butyl-2-nitroso-*N*,*N*-dimethylanilin, 4-*tert*-Butyl-2-nitroso-*N*,*N*-diethylanilin, 4-*tert*-Butyl-2-nitroso-*N*,*N*-diphenylanilin, 4-Nitroso-*N*-(1,3-dimethylbutyl)-anilin, 2-Nitroso-*N-*(1,3-dimethylbutyl)-anilin, 4-*tert*-Butyl-2-nitrosoanilin.

Erfindungsgemäß bevorzugte Hydrochinone sind jene der chemischen Struktur **(IX),** und erfindungsgemäß bevorzugte Benzochinone sind jene der chemischen Struktur **(X)** mit: 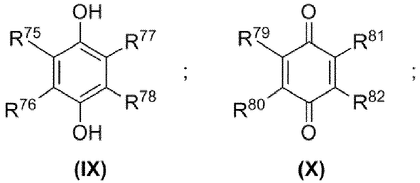 wobei R⁷⁵, R⁷⁶, R⁷⁷, R⁷⁸, R⁷⁹, R⁸⁰, R⁸¹, R⁸² unabhängig voneinander jeweils ausgewählt sind aus der Gruppe bestehend aus Wasserstoff, Alkylgruppe mit 1 bis 18 Kohlenstoffatomen, -F, -Cl, -Br, -I, -OR⁸³, wobei R⁸³ ausgewählt ist aus der Gruppe bestehend aus Wasserstoff, Alkylgruppe mit 1 bis 18 Kohlenstoffatomen, Arylgruppe mit 6 bis 14 Kohlenstoffatomen;
wobei die beiden Reste R⁷⁵ und R⁷⁶ außerdem auch einen aromatischen oder aliphatischen Ring miteinander bilden können, welcher auch Heteroatome ausgewählt aus O, S, N aufweisen kann;
wobei die beiden Reste R⁷⁷ und R⁷⁸ außerdem auch einen aromatischen oder aliphatischen Ring miteinander bilden können, welcher auch Heteroatome ausgewählt aus O, S, N aufweisen kann;
wobei die beiden Reste R⁷⁹ und R⁸⁰ außerdem auch einen aromatischen oder aliphatischen Ring miteinander bilden können, welcher auch Heteroatome ausgewählt aus O, S, N aufweisen kann;
wobei die beiden Reste R⁸¹ und R⁸² außerdem auch einen aromatischen oder aliphatischen Ring miteinander bilden können, welcher auch Heteroatome ausgewählt aus O, S, N aufweisen kann.

Erfindungsgemäß bevorzugte Hydrochinone sind jene der vorgenannten chemischen Struktur **(IX),** bei denen R⁷⁵ = R⁷⁶ = R⁷⁷ = Wasserstoff und R⁷⁸ eine Alkylgruppe mit 1 bis 18, insbesondere 1 bis 6, Kohlenstoffatomen, bevorzugt *tert*-Butyl oder Methyl, noch bevorzugter Methyl ist.

Andere erfindungsgemäß bevorzugte Hydrochinone sind jene der vorgenannten chemischen Struktur **(IX),** bei denen R⁷⁵ = R⁷⁶ = Wasserstoff und R⁷⁷, R⁷⁸ unabhängig voneinander jeweils eine Alkylgruppe mit 1 bis 18, insbesondere 1 bis 6, Kohlenstoffatomen, bevorzugt *tert*-Butyl oder Methyl, noch bevorzugter Methyl ist.

Andere erfindungsgemäß bevorzugte Hydrochinone sind jene der vorgenannten chemischen Struktur **(IX),** bei denen R⁷⁵ = R⁷⁷ = Wasserstoff und R⁷⁶, R⁷⁸ unabhängig voneinander jeweils eine Alkylgruppe mit 1 bis 18, insbesondere 1 bis 6, Kohlenstoffatomen, bevorzugt *tert*-Butyl oder Methyl, noch bevorzugter Methyl ist.

Andere erfindungsgemäß bevorzugte Hydrochinone sind jene der vorgenannten chemischen Struktur **(IX),** bei denen R⁷⁵ = R⁷⁸ = Wasserstoff und R⁷⁶, R⁷⁷ unabhängig voneinander jeweils eine Alkylgruppe mit 1 bis 18, insbesondere 1 bis 6, Kohlenstoffatomen, bevorzugt *tert*-Butyl oder Methyl, noch bevorzugter Methyl ist.

Ein anderes erfindungsgemäß bevorzugtes Hydrochinon ist jenes der vorgenannten chemischen Struktur **(IX),** bei der R⁷⁵ = R⁷⁶ = R⁷⁷ = R⁷⁸ = Wasserstoff ist.

Erfindungsgemäß bevorzugte Benzochinone sind jene der vorgenannten chemischen Struktur **(X),** bei denen R⁷⁹ = R⁸⁰ = R⁸¹ = Wasserstoff und R⁸² eine Alkylgruppe mit 1 bis 18, insbesondere 1 bis 6, Kohlenstoffatomen, bevorzugt *tert*-Butyl oder Methyl, noch bevorzugter Methyl ist.

Andere erfindungsgemäß bevorzugte Benzochinone sind jene der vorgenannten chemischen Struktur **(X),** bei denen R⁷⁹ = R⁸⁰ = Wasserstoff und R⁸¹, R⁸² unabhängig voneinander jeweils eine Alkylgruppe mit 1 bis 18, insbesondere 1 bis 6, Kohlenstoffatomen, bevorzugt *tert*-Butyl oder Methyl, noch bevorzugter Methyl ist.

Andere erfindungsgemäß bevorzugte Benzochinone sind jene der vorgenannten chemischen Struktur **(X),** bei denen R⁷⁹ = R⁸¹ = Wasserstoff und R⁸⁰, R⁸² unabhängig voneinander jeweils eine Alkylgruppe mit 1 bis 18, insbesondere 1 bis 6, Kohlenstoffatomen, bevorzugt *tert*-Butyl oder Methyl, noch bevorzugter Methyl ist.

Andere erfindungsgemäß bevorzugte Benzochinone sind jene der vorgenannten chemischen Struktur **(X),** bei denen R⁷⁹ = R⁸² = Wasserstoff und R⁸⁰, R⁸¹ unabhängig voneinander jeweils eine Alkylgruppe mit 1 bis 18, insbesondere 1 bis 6, Kohlenstoffatomen, bevorzugt *tert*-Butyl oder Methyl, noch bevorzugter Methyl ist.

Ein anderes erfindungsgemäß bevorzugtes Benzochinon ist jenes der vorgenannten chemischen Struktur **(X),** bei der R⁷⁹ = R⁸⁰= R⁸¹ = R⁸² = Wasserstoff ist.

Phenothiazine, die erfindungsgemäß bevorzugt eingesetzt werden, sind jene der chemischen Struktur **(XI)** mit: 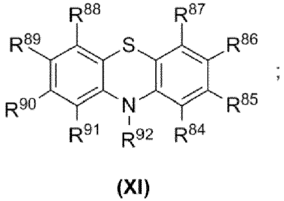 wobei R⁸⁴, R⁸⁵, R⁸⁶, R⁸⁷, R⁸⁸, R⁸⁹, R⁹⁰, R⁹¹, R⁹² unabhängig voneinander jeweils ausgewählt aus der Gruppe bestehend aus Wasserstoff, Alkylgruppe mit 1 bis 18 Kohlenstoffatomen sind.

In einer erfindungsgemäß bevorzugten Ausführungsform ist in der chemischen Struktur **(XI)** R⁹² = Wasserstoff oder Alkylgruppe mit 1 bis 18 Kohlenstoffatomen, einer der Reste R⁸⁴, R⁸⁵, R⁸⁶, R⁸⁷, R⁸⁸, R⁸⁹, R⁹⁰, R⁹¹ ist auch Wasserstoff oder Alkylgruppe mit 1 bis 18 Kohlenstoffatomen, während die übrigen der Reste R⁸⁴, R⁸⁵, R⁸⁶, R⁸⁷, R⁸⁸, R⁸⁹, R⁹⁰, R⁹¹ jeweils Wasserstoff sind.

Noch bevorzugter sind R⁸⁴ = R⁸⁶ = R⁸⁷ = R⁸⁸ = R⁸⁹ = R⁹⁰ = R⁹¹ = Wasserstoff, während R⁸⁵ und R⁹² unabhängig voneinander jeweils ausgewählt aus der Gruppe bestehend aus Wasserstoff, Alkylgruppe mit 1 bis 6 Kohlenstoffatomen sind.

Phenolische Antioxidantien, die erfindungsgemäß bevorzugt eingesetzt werden, sind jene der chemischen Struktur **(XII)** mit: 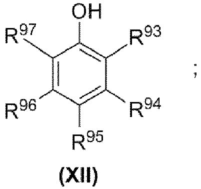 wobei
R⁹³, R⁹⁴, R⁹⁵, R⁹⁶, R⁹⁷ unabhängig voneinander jeweils ausgewählt aus der Gruppe bestehend aus Wasserstoff, Alkylgruppe mit 1 bis 18 Kohlenstoffatomen, Arylgruppe mit 6 bis 14 Kohlenstoffatomen, Benzylgruppe sind, und wobei R⁹³, R⁹⁴, R⁹⁶, R⁹⁷ auch jeweils eine Hydroxygruppe sein können;
wobei die Arylgruppe mit 6 bis 14 Kohlenstoffatomen und die Benzylgruppe ihrerseits jeweils substituiert sein können mit mindestens einem Rest ausgewählt aus der Gruppe bestehend aus Hydroxygruppe, Halogen, Alkylgruppe mit 1 bis 6 Kohlenstoffatomen.

Bevorzugt sind erfindungsgemäß R⁹³, R⁹⁴, R⁹⁵, R⁹⁶, R⁹⁷ in der chemischen Struktur **(XII)** wie folgt ausgewählt:
R⁹⁴ = R⁹⁶ = Wasserstoff; R⁹³ and R⁹⁷ sind unabhängig voneinander jeweils ausgewählt aus der Gruppe bestehend aus Hydroxy, Alkylgruppe mit 1 bis 6 Kohlenstoffatomen, bevorzugt aus der Gruppe bestehend aus Hydroxy, Methyl, *tert*-Butyl; R⁹⁵ ist ausgewählt aus der Gruppe bestehend aus Alkylgruppe mit 1 bis 6 Kohlensotffatomen, Benzylgruppe, wobei mindestens ein Wasserstoffrest der Benzylgruppe durch einen Rest ausgewählt aus der Gruppe bestehend aus Hydroxy, Alkygruppe mit 1 bis 6 Kohlenstoffatomen ersetzt sein kann.

Ganz besonders bevorzugte phenolische Antioxidantien sind jene der chemischen Strukturen **(XII-A), (XII-B), (XII-C), (XII-D)** mit:

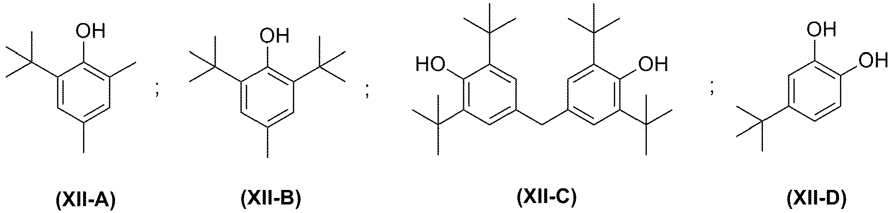

Die Struktur **(XII-B)** ist dabei 2,6-Di-*tert*-butyl-*p*-kresol ("BHT").

Erfindungsgemäß bevorzugte *N*,*N*'-Dialkyl-*p*-phenylendiamine sind jene der chemischen Struktur **(XIII).** Erfindungsgemäß bevorzugte *N*,*N*'-Dialkyl-*p*-benzochinondümide sind jene der chemischen Struktur **(XIV)** mit: 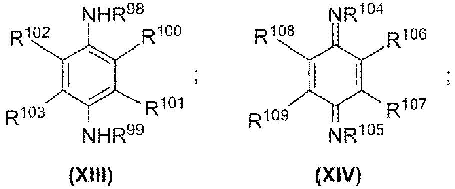 wobei
R⁹⁸, R⁹⁹, R¹⁰⁴, R¹⁰⁵ unabhängig voneinander jeweils eine Alkylgruppe mit 1 bis 18 Kohlenstoffatomen ist;
R¹⁰⁰, R¹⁰¹, R¹⁰², R¹⁰³ R¹⁰⁶, R¹⁰⁷, R¹⁰⁸, R¹⁰⁹ unabhängig voneinander jeweils ausgewählt aus der Gruppe bestehend aus Wasserstoff, Alkylgruppe mit 1 bis 18 Kohlenstoffatomen, -F, -Cl, -Br, -I, -OR¹¹⁰ sind; wobei R¹¹⁰ ausgewählt aus der Gruppe bestehend aus Wasserstoff, Alkylgruppe mit 1 bis 18 Kohlenstoffatomen, Arylgruppe mit 6 bis 14 Kohlenstoffatomen ist;
wobei die beiden Reste R¹⁰⁰ und R¹⁰¹ außerdem auch einen aromatischen oder aliphatischen Ring miteinander bilden können, welcher auch Heteroatome ausgewählt aus O, S, N aufweisen kann;
wobei die beiden Reste R¹⁰² und R¹⁰³ außerdem auch einen aromatischen oder aliphatischen Ring miteinander bilden können, welcher auch Heteroatome ausgewählt aus O, S, N aufweisen kann;
wobei die beiden Reste R¹⁰⁶ und R¹⁰⁷ außerdem auch einen aromatischen oder aliphatischen Ring miteinander bilden können, welcher auch Heteroatome ausgewählt aus O, S, N aufweisen kann;
wobei die beiden Reste R¹⁰⁸ und R¹⁰⁹ außerdem auch einen aromatischen oder aliphatischen Ring miteinander bilden können, welcher auch Heteroatome ausgewählt aus O, S, N aufweisen kann.

In einer erfindungsgemäß bevorzugten Ausführungsform ist in der chemischen Struktur **(XIII),** R¹⁰⁰ = R¹⁰¹ = R¹⁰² = R¹⁰³ = Wasserstoff, während R⁹⁸ = R⁹⁹ = Alkylgruppe mit 1 bis 18 Kohlenstoffatomen. Noch bevorzugter ist in der chemischen Struktur **(XIII),** R¹⁰⁰ = R¹⁰¹ = R¹⁰² = R¹⁰³ = Wasserstoff, während R⁹⁸ = R⁹⁹ = Alkylgruppe mit 1 bis 12, insbesondere 1 bis 6, Kohlenstoffatomen.

In einer erfindungsgemäß bevorzugten Ausführungsform ist in der chemischen Struktur **(XIV),** R¹⁰⁶ = R¹⁰⁷ = R¹⁰⁸ = R¹⁰⁹ = Wasserstoff, während R¹⁰⁴ = R¹⁰⁵ = Alkylgruppe mit 1 bis 18 Kohlenstoffatomen. Noch bevorzugter ist in der chemischen Struktur **(XIV),** R¹⁰⁶ = R¹⁰⁷ = R¹⁰⁸ = R¹⁰⁹ = Wasserstoff, während R¹⁰⁴ = R¹⁰⁵ = Alkylgruppe mit 1 bis 12, insbesondere 1 bis 6, Kohlenstoffatomen.

In einem zweiten Aspekt betrifft die vorliegende Erfindung eine Zusammensetzung **(AB*)** umfassend
**(A)** mindestens eine Verbindung der chemischen Struktur **(I)** mit 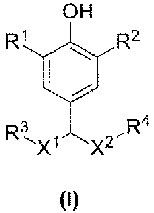
   wobei X¹ und X² unabhängig voneinander jeweils S oder O ist;
   wobei R¹, R² unabhängig voneinander jeweils ausgewählt aus der Gruppe bestehend aus Wasserstoff, Alkylgruppe mit 1 bis 18 Kohlenstoffatomen, Cycloalkylgruppe mit 3 bis 15 Kohlenstoffatomen, Arylgruppe mit 6 bis 14 Kohlenstoffatomen sind;
   wobei R³, R⁴ unabhängig voneinander jeweils ausgewählt aus der Gruppe bestehend aus
      -(CH₂)ₐ[O(CH₂)_{b}]_{c}R⁵, wobei R⁵ = H oder OH; a, c unabhängig voneinander jeweils eine ganze Zahl aus dem Bereich 1 bis 10 ist; und b = 1, 2, 3 oder 4 ist;
      Alkylgruppe mit 1 bis 18 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR⁶, -OR⁷, -SR⁸, -NR⁹R¹⁰, -CN, Arylgruppe mit 6 bis 14 Kohlenstoffatomen substituiert sein kann;
      Cycloalkylgruppe mit 3 bis 15 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹¹, -OR¹², -SR¹³, -NR¹⁴R¹⁵, -CN substituiert sein kann;
      Arylgruppe mit 6 bis 14 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹⁶, -OR¹⁷, -SR¹⁸, -NR¹⁹R²⁰, -CN, Alkylgruppe mit 1 bis 6 Kohlenstoffatomen substituiert sein kann;
      Heteroarylgruppe mit 1 bis 10 Kohlenstoffatomen, bei welcher mindestens ein an ein Kohlenstoffatom gebundener Wasserstoffrest durch einen Rest ausgewählt aus der Gruppe bestehend
      aus -F, -Cl, -Br, -I, -NH, -OH, -SH, -NHR²¹, -OR²², -SR²³, -NR²⁴R²⁵, -CN, Alkylgruppe mit 1 bis 6 Kohlenstoffatomen substituiert sein kann;
      Heterocycloalkylgruppe mit 2 bis 14 Kohlenstoffatomen, bei welcher mindestens ein an ein Kohlenstoffatom gebundener Wasserstoffrest durch einen Rest ausgewählt aus der Gruppe bestehend
         aus -F, -Cl, -Br, -I, -NH, -OH, -SH, -NHR²⁶, -OR²⁷, -SR²⁸, -NR²⁹R³⁰, -CN substituiert sein kann;
         wobei R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ unabhängig voneinander jeweils aus der Gruppe bestehend aus Alkylgruppe mit 1 bis 18 Kohlenstoffatomen, Cycloalkylgruppe mit 3 bis 15 Kohlenstoffatomen ausgewählt sind;
      sind;
      und
**(B)** mindestens ein olefinisch ungesättigtes Monomer ist; wobei die Zusammensetzung **(AB*)** dadurch gekennzeichnet ist, dass das Gesamtgewicht aller von der Zusammensetzung **(AB*)** umfassten Verbindungen der Struktur **(I)** im Bereich von 1 ppm (m/m) bis 100.000 ppm (m/m) liegt, bezogen auf das Gesamtgewicht aller von der Zusammensetzung **(AB*)** umfassten olefinisch ungesättigten Monomere,
   und wobei das olefinisch ungesättigte Monomer ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid ist.

In einer bevorzugten Ausführungsform der Zusammensetzung **(AB*)** gemäß des zweiten Aspekts der Erfindung sind X¹ = X² = O; und R¹, R² sind unabhängig voneinander jeweils ausgewählt aus der Gruppe bestehend aus Wasserstoff, Alkylgruppe mit 1 bis 18 Kohlenstoffatomen; und R³, R⁴ sind unabhängig voneinander jeweils ausgewählt aus der Gruppe bestehend aus Alkylgruppe mit 1 bis 18 Kohlenstoffatomen, Arylgruppe mit 6 bis 14 Kohlenstoffatomen, Benzyl. Noch bevorzugter sind in der Zusammensetzung **(AB*)** gemäß des zweiten Aspekt der Erfindung X¹ = X² = O; R¹ und R² sind gleich und jeweils ausgewählt aus der Gruppe bestehend aus Methyl, *n*-Propyl, *iso*-Propyl, *tert*-Butyl sind und gleichzeitig sind auch R³ und R⁴ gleich und jeweils eine Alkylgruppe mit 1 bis 6 Kohlenstoffatomen oder jeweils Phenyl oder jeweils Benzyl.

Am bevorzugtesten sind in der Zusammensetzung **(AB*)** gemäß des zweiten Aspekt der Erfindung X¹ = X² = O; R¹ = *tert*-Butyl; R² = *tert*-Butyl; und R³ und R⁴ sind gleich und jeweils ausgewählt aus der Gruppe bestehend aus Methyl, Ethyl, *n*-Propyl, *iso*-Propyl, *n*-Butyl, *n*-Pentyl, *n*-Hexyl, Phenyl, Benzyl.

In einer ersten ganz besonders bevorzugten Ausführungsform der Zusammensetzung **(AB*)** gemäß des zweiten Aspekts der Erfindung weist die Verbindung der Struktur **(I)** die vorgenannte Struktur **(I-1)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer zweiten ganz besonders bevorzugten Ausführungsform der Zusammensetzung **(AB*)** gemäß des zweiten Aspekts der Erfindung weist die Verbindung der Struktur **(I)** die vorgenannte Struktur **(I-2)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer dritten ganz besonders bevorzugten Ausführungsform der Zusammensetzung **(AB*)** gemäß des zweiten Aspekts der Erfindung weist die Verbindung der Struktur **(I)** die vorgenannte Struktur **(I-3)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer vierten ganz besonders bevorzugten Ausführungsform der Zusammensetzung **(AB*)** gemäß des zweiten Aspekts der Erfindung weist die Verbindung der Struktur **(I)** die vorgenannte Struktur **(I-4)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer fünften ganz besonders bevorzugten Ausführungsform der Zusammensetzung **(AB*)** gemäß des zweiten Aspekts der Erfindung weist die Verbindung der Struktur **(I)** die vorgenannte Struktur **(I-5)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer sechsten ganz besonders bevorzugten Ausführungsform der Zusammensetzung **(AB*)** gemäß des zweiten Aspekts der Erfindung weist die Verbindung der Struktur **(I)** die vorgenannte Struktur **(I-6)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer siebten ganz besonders bevorzugten Ausführungsform der Zusammensetzung **(AB*)** gemäß des zweiten Aspekts der Erfindung weist die Verbindung der Struktur **(I)** die vorgenannte Struktur **(I-7)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer achten ganz besonders bevorzugten Ausführungsform der Zusammensetzung **(AB*)** gemäß des zweiten Aspekts der Erfindung weist die Verbindung der Struktur **(I)** die vorgenannte Struktur **(I-8)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In einer neunten ganz besonders bevorzugten Ausführungsform der Zusammensetzung **(AB*)** gemäß des zweiten Aspekts der Erfindung weist die Verbindung der Struktur **(I)** die vorgenannte Struktur **(I-9)** auf und das olefinisch ungesättigte Monomer ist ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid; insbesondere ist das olefinisch ungesättigte Monomer Styrol.

In der erfindungsgemäßen Zusammensetzung **(AB*)** liegt **(A)** in einer effektiven Menge vor. Somit liegt das Gesamtgewicht aller von der Zusammensetzung **(AB*)** umfassten Verbindungen der Struktur **(I)** im Bereich von 1 ppm (m/m) bis 100.000 ppm (m/m), bezogen auf das Gesamtgewicht aller von der Zusammensetzung **(AB*)** umfassten olefinisch ungesättigten Monomere.
Insbesondere liegt das Gesamtgewicht aller von der Zusammensetzung **(AB*)** umfassten Verbindungen der Struktur **(I)** im Bereich von 1 ppm (m/m) bis 50000 ppm (m/m),bevorzugt im Bereich von 10 ppm bis 10000 ppm (m/m), bevorzugter im Bereich von 100 ppm bis 5000 ppm (m/m), noch bevorzugter im Bereich von 200 ppm bis 500 ppm (m/m), jeweils bezogen auf das Gesamtgewicht aller von der Zusammensetzung **(AB*)** umfassten olefinisch ungesättigten Monomere.

Die erfindungsgemäß Zusammensetzung **(AB*)** umfasst bevorzugt auch **(C)** mindestens ein Lösungsmittel. Als Lösungsmittel im erfindungsgemäßen Verfahren eignen sich alle Stoffe, in denen **(A)** im gewünschten Konzentrationsbereich löslich ist und die mit **(A)** verträglich sind, insbesondere unpolare, bevorzugt unpolare aromatische oder aliphatische Lösungsmittel. Bevorzugt ist das Lösungsmittel ausgewählt aus der Gruppe bestehend aus Benzol, einfach oder mehrfach alkylierten Aromaten, Alkanen mit einer Kohlenstoffzahl von 6 bis 15, Cycloalkanen mit einer Kohlenstoffzahl von 6 bis 15, hochsiedenden Kohlenwasserstoffschnitten, Ethern mit einer Kohlenstoffzahl von 6 bis 15, Estern mit einer Kohlenstoffzahl von 6 bis 15.

Noch bevorzugter ist das Lösungsmittel in der Zusammensetzung **(AB*)** ausgewählt aus der Gruppe bestehend aus Benzol, Toluol, Ethylbenzol, Xylol, Styrol, hochsiedende aromatische Kohlenwasserstoffschnitte. Besonders bevorzugt ist das Lösungsmittel in der Zusammensetzung **(AB*)** ausgewählt aus der Gruppe bestehend aus Toluol, Ethylbenzol, Xylol und Styrol.

Umfasst die erfindungsgemäße Zusammensetzung **(AB*)** auch **(C)**, so liegt bevorzugt in der resultierenden Lösung **(AB*)** das Verhältnis (m/m) des Gesamtgewichts aller von der Zusammensetzung **(AB*)** umfassten Verbindungen der Struktur **(I)** zum Gesamtgewicht aller von der Zusammensetzung **(AB*)** umfassten Lösungsmittel im Bereich von 1 : 1000 bis 100 : 1, bevorzugter im Bereich von 1 : 100 bis 10 : 1, noch bevorzugter im Bereich von 1 : 10 bis 3 : 1.

In einer weiteren bevorzugten Ausführungsform umfasst die Zusammensetzung **(AB*)** auch **(D)** mindestens eine Verbindung ausgewählt aus der Gruppe bestehend aus Hydroxylaminen, Oximen, sterisch gehinderten *N*-Oxyl-Verbindungen, Chinonmethiden, Nitroaromaten, Nitrosoaromaten, Benzochinonen, Hydrochinonen, Phenothiazinen, phenolischen Antioxidantien, *N*,*N*'-Dialkyl-*p*-phenylendiaminen, *N*,*N*'-Dialkyl-*p*-benzochinondiimiden.

**(D)** ist dabei insbesondere mindestens eine Verbindung ausgewählt aus der Gruppe bestehend aus Hydroxylaminen, sterisch gehinderten *N*-Oxyl-Verbindungen, Chinonmethiden, Nitroaromaten, Nitrosoaromaten, Benzochinonen, Hydrochinonen, Phenothiazinen, phenolischen Antioxidantien, *N*,*N*'-Dialkyl-*p*-phenylendiaminen, *N*,*N*'-Dialkyl-*p*-benzochinondiimiden.

**(D)** ist dabei bevorzugt mindestens eine Verbindung ausgewählt aus der Gruppe bestehend aus sterisch gehinderten *N*-Oxyl-Verbindungen, Chinonmethiden, Nitroaromaten.

Die bevorzugt von der Zusammensetzung **(AB*)** in diesen Ausführungsformen umfassten Hydroxylamine, Oxime, sterisch gehinderten *N*-Oxyl-Verbindungen, Chinonmethide, Nitroaromaten, Nitrosoaromaten, Benzochinone, Hydrochinone, Phenothiazine, phenolischen Antioxidantien, *N*,*N*'-Dialkyl-*p*-phenylendiamine, *N*,*N*'-Dialkyl-*p*-benzochinondiimide sind im Kontext des erfindungsgemäßen Verfahrens gemäß des ersten Aspektes der vorliegenden Erfindung vorstehend beschrieben.

Es handelt sich dabei insbesondere um die dort beschriebenen Nitroaromaten, Nitrosoaromaten, die Verbindungen der chemischen Struktur **(II-A),** die Verbindungen der chemischen Struktur **(II-B),** die Verbindungen der chemischen Struktur **(III),** die Verbindungen der chemischen Struktur **(IV),** die Verbindungen der chemischen Struktur **(V)**, die Verbindungen der chemischen Struktur **(VI),** die Verbindungen der chemischen Struktur **(VII),** die Verbindungen der chemischen Struktur **(VIII),** die Verbindungen der chemischen Struktur **(IX),** die Verbindungen der chemischen Struktur **(X)**, die Verbindungen der chemischen Struktur **(XI),** die Verbindungen der chemischen Struktur **(XII)** [und im Falle von **(XII)** insbesondere die Verbindung der chemischen Struktur **(XII-A),** die Verbindung der chemischen Struktur **(XII-B)**, die Verbindung der chemischen Struktur **(XII-C),** die Verbindung der chemischen Struktur **(XII-D)]**, die Verbindungen der chemischen Struktur **(XIII)**, die Verbindungen der chemischen Struktur **(XIV)** mit den vorstehend angegebenen Bedeutungen.

Die nachfolgenden Beispiele sollen die Erfindung näher erläutern, ohne dass die Erfindung auf diese Ausführungsformen beschränkt sein soll.

### Beispiele

### 1. Vergleichsbeispiele V1 bis V3 und erfindungsgemäßes Beispiel E1 (110 °C)

Käuflich erwerbbares, stabilisiertes Styrol wurde bei einem reduziertem Druck von 95 mbar und einer Sumpftemperatur von 75 °C in einer inerten Stickstoffatmosphäre von dem Stabilisator *tert*-Butyl-1,2-hydroxybenzol ("TBC") befreit. Die Versuchsapparatur, die aus einem Vierhalskolben, der mit einem Thermometer, einem Rückflusskühler, einem Septum und einem KPG-Rührer versehen war, bestand, wurde gründlich mit Stickstoff gespült, um eine sauerstofffreie Atmosphäre zu erhalten. 300 g des unstabilisierten Styrols wurden in den Dreihalskolben gegeben und ohne weiteres Additiv (Vergleichsbeispiel V1); mit 100 ppm eines phenolischen Cycloacetals mit der chemischen Struktur **(XV)** (Vergleichsbeispiel V2) mit: 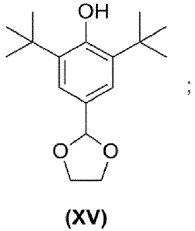 mit 100 ppm BHT [Struktur **(XII-B);** Vergleichsbeispiel V3] bzw. mit 100 ppm eines phenolischen Acetals der chemischen Struktur **(I-1)** (erfindungsgemäßes Beispiel E1) versetzt. Die Zugabe des jeweiligen Additivs erfolgte als Lösung in Styrol. "100 ppm" bedeuten dabei 100 mg Additiv pro 1 kg eingesetztem Styrol. Durch die ständige Stickstoffzufuhr über eine Glasfritte in die Styrol-Lösung wurde eine inerte Stickstoffatmosphäre über die gesamte Versuchsdauer gewährleistet. Die Styrol-Lösung wurd kräftig gerührt. Zum Start des Experiments wurde der Kolben in ein Ölbad, welches auf 110 °C vorgeheizt worden war, so weit eingetaucht, dass die stabilisierte Styrol-Lösung komplett eingetaucht war. Nach dem Eintauchen des Dreihalskolbens in das geheizte Ölbad wurde in regelmäßigen Abständen ca. 3 g der Styrol-Lösung über das Septum entnommen, genau ausgewogen und in 50 ml Methanol gegeben. Die Methanol-Mischung wurde für eine halbe Stunde bei Raumtemperatur gerührt. Das Methanol bewirkte die Ausfällung des während des Versuchs gebildeten Polystyrols. Dieses wurde durch Filtration über einen Glasfiltertiegel abgetrennt. Der Filterrückstand wurde mit 20 ml Methanol gewaschen und anschließend für mindestens 5 Stunden bei 110 °C getrocknet. Das im Glasfiltertiegel zurückgebliebene Polystyrol wurde nun ausgewogen. Aus dem ermittelten Wert und der Einwaage wurde der prozentuale Anteil an Polymer ermittelt. Dieser Polymergehalt wurde gegen die Reaktionszeit aufgetragen. Die Zeitdauer, in der sich ein Polymergehalt von 2 Gew.-% gebildet hat, und der Polymergehalt nach 60 Minuten wurden ermittelt. Die Ergebnisse sind in Tabelle 1 dargestellt.

**Tabelle 1 (Ölbadtemperatur 110 C; in V2, V3, E1 eingesetzte Menge des Additivs: 100 ppm)**

| Beispiel | Additiv | Zeitdauer bis 2 Gew.-% Polymergehalt vorliegt (*in min*) | Polymergehalt nach 60 Minuten *(in Gew.-%)* |
|---|---|---|---|
| V1 | - | 30 | 3.8 |
| V2 | **(XV)** | 41 | 3.3 |
| V3 | **(XII-B)** | 33 | 3.7 |
| E1 | **(I-1)** | 105 | 1.0 |

Tabelle 1 zeigt deutlich, dass die erfindungsgemäßen nicht cyclischen Acetale, hier durch das Methylderivat **(I-1)** repräsentiert, eine deutlich bessere Inhibierung des Styrolpolymerisation erlauben als die von Skurko *et al.* beschriebenen cyclischen Acetale, repräsentiert durch die Verbindung der chemischen Struktur **(XV).**

Die Tabelle 1 zeigt ebenfalls, dass die Angabe von Skurko *et al.* zutrifft und die inhibitorische Aktivität von BHT [Verbindung der chemischen Struktur **(XII-B)]** mit der der cyclischen Acetale vergleichbar ist.

### 2. Vergleichsbeispiel V4 und erfindungsgemäße Beispiele E2 bis E4 (120 °C)

Käuflich erwerbbares, stabilisiertes Styrol wurde bei einem reduziertem Druck von 95 mbar und einer Sumpftemperatur von 75 °C in einer inerten Stickstoffatmosphäre von dem Stabilisator *tert*-Butyl-1,2-hydroxybenzol ("TBC") befreit. Die Versuchsapparatur, die aus einem Vierhalskolben, der mit einem Thermometer, einem Rückflusskühler, einem Septum und einem KPG-Rührer versehen war, bestand, wurde gründlich mit Stickstoff gespült, um eine sauerstofffreie Atmosphäre zu erhalten. 300 g des unstabilisierten Styrols wurden in den Dreihalskolben gegeben und ohne weiteres Additiv (Vergleichsbeispiel V4);
mit 200 ppm eines phenolischen Acetals der chemischen Struktur **(I-1)** (erfindungsgemäßes Beispiel E2); mit 200 ppm eines phenolischen Acetals der chemischen Struktur **(I-2)** (erfindungsgemäßes Beispiel E3) bzw. mit 200 ppm eines phenolischen Acetals der chemischen Struktur **(I-3)** (erfindungsgemäßes Beispiel E4) versetzt. Die Zugabe des jeweiligen Additivs erfolgte als Lösung in Styrol. "200 ppm" bedeuten dabei 200 mg Additiv pro 1 kg eingesetztem Styrol. Durch die ständige Stickstoffzufuhr über eine Glasfritte in die Styrol-Lösung wurde eine inerte Stickstoffatmosphäre über die gesamte Versuchsdauer gewährleistet. Die Styrol-Lösung wurde kräftig gerührt. Zum Start des Experiments wurde der Kolben in ein Ölbad, welches auf 120 °C vorgeheizt worden war, so weit eingetaucht, dass die stabilisierte Styrol-Lösung komplett eingetaucht war. Nach dem Eintauchen des Dreihalskolbens in das geheizte Ölbad wurde in regelmäßigen Abständen ca. 3 g der Styrol-Lösung über das Septum entnommen, genau ausgewogen und in 50 ml Methanol zugegeben. Die Methanol-Mischung wurde für eine halbe Stunde bei Raumtemperatur gerührt. Das Methanol bewirkte die Ausfällung des während des Versuchs gebildeten Polystyrols. Dieses wurde durch Filtration über einen Glasfiltertiegel abgetrennt. Der Filterrückstand wurde mit 20 ml Methanol gewaschen und anschließend für mindestens 5 Stunden bei 110 °C getrocknet. Das im Glasfiltertiegel zurückgebliebene Polystyrol wurde nun ausgewogen. Aus dem ermittelten Wert und der Einwaage wurde der prozentuale Anteil an Polymer ermittelt. Dieser Polymergehalt wurde gegen die Reaktionszeit aufgetragen. Die Zeitdauer, in der sich ein Polymergehalt von 2 Gew.-% gebildet hat, und der Polymergehalt nach 60 Minuten wurden ermittelt. Die Ergebnisse sind in Tabelle 2 dargestellt.

**Tabelle 2 (Ölbadtemperatur 120 °C; in E2, E3, E4 war die eingesetzte Menge des Additivs: 200 ppm)**

| Beispiel | Additiv | Zeitdauer bis 2 Gew.-% Polymergehalt vorliegt (*in min*) | Polymergehalt nach 60 Minuten *(in Gew.-%)* |
|---|---|---|---|
| V4 | - | 18 | 10.4 |
| E2 | **(I-1**) | 80 | 1.3 |
| E3 | **(I-2)** | 23 | 6.5 |
| E4 | (**I**-**3**) | 20 | 7.4 |

Aus Tabelle 2 folgt, dass die erfindungsgemäßen nicht cyclischen Acetale, hier durch das Methylderivat **(I-1),** das Ethylderivat **(I-2)** und das *n*-Propylderivat **(I-3)** repräsentiert, auch bei höheren Temperaturen von 120 °C einen deutlichen Inhibierungseffekt gegenüber der Blindprobe V4 zeigen.

### 3. Vergleichsbeispiel V5 und erfindungsgemäße Beispiele E5, E6, E7 (150 °C)

Käuflich erwerbbares, stabilisiertes Styrol wurde bei einem reduziertem Druck von 95 mbar und einer Sumpftemperatur von 75 °C in einer inerten Stickstoffatmosphäre von dem Stabilisator *tert*-Butyl-1,2-hydroxybenzol ("TBC") befreit. Die Versuchsapparatur, die aus einem Vierhalskolben, der mit einem Thermometer, einem Rückflusskühler, einem Septum und einem KPG-Rührer versehen war, bestand, wurde gründlich mit Stickstoff gespült, um eine sauerstofffreie Atmosphäre zu erhalten. 300 g des unstabilisierten Styrols wurden in den Dreihalskolben gegeben und ohne weiteres Additiv (Vergleichsbeispiel V5); mit 500 ppm eines phenolischen Acetals der chemischen Struktur **(I-1)** (erfindungsgemäßes Beispiel E5); mit 500 ppm eines phenolischen Acetals der chemischen Struktur **(I-2)** (erfindungsgemäßes Beispiel E6) bzw. mit 500 ppm eines phenolischen Acetals der chemischen Struktur **(I-3)** (erfindungsgemäßes Beispiel E7) versetzt. Die Zugabe des jeweiligen Additivs erfolgte als Lösung in Styrol. "500 ppm" bedeuten dabei 500 mg Additiv pro 1 kg eingesetztem Styrol. Durch die ständige Stickstoffzufuhr über eine Glasfritte in die Styrol-Lösung wurde eine inerte Stickstoffatmosphäre über die gesamte Versuchsdauer gewährleistet. Die Styrol-Lösung wurde kräftig gerührt. Zum Start des Experiments wurde der Kolben in ein Ölbad, welches auf 150 °C vorgeheizt worden war, so weit eingetaucht, dass die stabilisierte Styrol-Lösung komplett eingetaucht war. Nach dem Eintauchen des Dreihalskolbens in das geheizte Ölbad wurde in regelmäßigen Abständen ca. 3 g der Styrol-Lösung über das Septum entnommen, genau ausgewogen und in 50 ml Methanol gegeben. Die Methanol-Mischung wurde für eine halbe Stunde bei Raumtemperatur gerührt. Das Methanol bewirkte die Ausfällung des während des Versuchs gebildeten Polystyrols. Dieses wurde durch Filtration über einen Glasfiltertiegel abgetrennt. Der Filterrückstand wurde mit 20 ml Methanol gewaschen und anschließend für mindestens 5 Stunden bei 110 °C getrocknet. Das im Glasfiltertiegel zurückgebliebene Polystyrol wurde nun ausgewogen. Aus dem ermittelten Wert und der Einwaage wurde der prozentuale Anteil an Polymer ermittelt. Dieser Polymergehalt wurde gegen die Reaktionszeit aufgetragen. Die Zeitdauer, in der sich ein Polymergehalt von 2 Gew.-% gebildet hat, und der Polymergehalt nach 15 Minuten wurden ermittelt. Die Ergebnisse sind in Tabelle 3 dargestellt.

**Tabelle 3 (Ölbadtemperatur 150 °C; in E5, E6, E7 war die eingesetzte Menge des Additivs: 500 ppm)**

| Beispiel | Additiv | Zeitdauer bis 2 Gew.-% Polymergehalt vorliegt (*in min*) | Polymergehalt nach 15 Minuten *(in Gew.-%)* |
|---|---|---|---|
| V5 | - | 0.8 | 35.9 |
| E5 | **(I-1)** | 18.5 | 1.2 |
| E6 | **(I-2)** | 16.8 | 1.8 |
| E7 | **(I-3)** | 2 | 15.0 |

Aus Tabelle 3 folgt, dass die erfindungsgemäßen nicht cyclischen Acetale, hier durch das Methylderivat **(I-1),** das Ethylderivat **(I-2)** und das *n*-Propylderivat **(I-3)** repräsentiert, auch bei höheren Temperaturen von 150 °C einen deutlichen Inhibierungseffekt gegenüber der Blindprobe V5 zeigen.

## Patentansprüche

1. Verfahren zur Inhibierung der Polymerisation von **(B)**, **dadurch gekennzeichnet, dass (A)** mit **(B)** vermischt und dadurch eine Zusammensetzung **(AB)** erhalten wird, wobei
**(A)** mindestens eine Verbindung der chemischen Struktur **(I)** ist mit 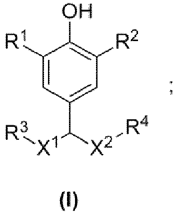
wobei X¹ und X² unabhängig voneinander jeweils S oder O ist;
wobei R¹, R² unabhängig voneinander jeweils ausgewählt aus der Gruppe bestehend aus Wasserstoff, Alkylgruppe mit 1 bis 18 Kohlenstoffatomen, Cycloalkylgruppe mit 3 bis 15 Kohlenstoffatomen, Arylgruppe mit 6 bis 14 Kohlenstoffatomen sind;
wobei R³, R⁴ unabhängig voneinander jeweils ausgewählt aus der Gruppe bestehend aus
-(CH₂)ₐ[O(CH₂)_{b}]_{c}R⁵, wobei R⁵ = H oder OH; a, c unabhängig voneinander jeweils eine ganze Zahl aus dem Bereich 1 bis 10 ist; und b = 1, 2, 3 oder 4 ist;
Alkylgruppe mit 1 bis 18 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR⁶, -OR⁷, -SR⁸, -NR⁹R¹⁰, -CN, Arylgruppe mit 6 bis 14 Kohlenstoffatomen substituiert sein kann;
Cycloalkylgruppe mit 3 bis 15 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹¹, -OR¹², -SR¹³, -NR¹⁴R¹⁵, -CN substituiert sein kann;
Arylgruppe mit 6 bis 14 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹⁶, -OR¹⁷, -SR¹⁸, -NR¹⁹R²⁰, -CN, Alkylgruppe mit 1 bis 6 Kohlenstoffatomen substituiert sein kann;
Heteroarylgruppe mit 1 bis 10 Kohlenstoffatomen, bei welcher mindestens ein an ein Kohlenstoffatom gebundener Wasserstoffrest durch einen Rest ausgewählt aus der Gruppe bestehend
aus -F, -Cl, -Br, -I, -NH, -OH, -SH, -NHR²¹, -OR²², -SR²³, -NR²⁴R²⁵, -CN, Alkylgruppe mit 1 bis 6 Kohlenstoffatomen substituiert sein kann;
Heterocycloalkylgruppe mit 2 bis 14 Kohlenstoffatomen, bei welcher mindestens ein an ein Kohlenstoffatom gebundener Wasserstoffrest durch einen Rest ausgewählt aus der Gruppe bestehend
aus -F, -Cl, -Br, -I, -NH, -OH, -SH, -NHR²⁶, -OR²⁷, -SR²⁸, -NR²⁹R³⁰, -CN substituiert sein kann;
wobei R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ unabhängig voneinander jeweils aus der Gruppe bestehend aus Alkylgruppe mit 1 bis 18 Kohlenstoffatomen, Cycloalkylgruppe mit 3 bis 15 Kohlenstoffatomen ausgewählt sind;
sind;
und wobei
**(B)** mindestens ein olefinisch ungesättigtes Monomer ist,
wobei **(A)** in einer solchen Menge eingesetzt wird, dass das Gesamtgewicht aller im Verfahren eingesetzten Verbindungen der Struktur **(I)** im Bereich von 1 ppm (m/m) bis 100000 ppm (m/m) liegt, jeweils bezogen auf das Gesamtgewicht aller im Verfahren eingesetzten olefinisch ungesättigten Monomere,
und wobei das olefinisch ungesättigte Monomer ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid ist.

2. Verfahren nach Anspruch 1, wobei
X¹ = X² = O;
R¹, R² unabhängig voneinander jeweils ausgewählt aus der Gruppe bestehend aus Wasserstoff, Alkylgruppe mit 1 bis 18 Kohlenstoffatomen sind;
und R³, R⁴ unabhängig voneinander jeweils ausgewählt aus der Gruppe bestehend aus Alkylgruppe mit 1 bis 18 Kohlenstoffatomen, Arylgruppe mit 6 bis 14 Kohlenstoffatomen, Benzyl sind.

3. Verfahren nach Anspruch 2, wobei R¹ und R² gleich sind und jeweils ausgewählt aus der Gruppe bestehend aus Methyl, *n*-Propyl, *iso*-Propyl, *tert*-Butyl sind;
und wobei R³ und R⁴ gleich sind und jeweils eine Alkylgruppe mit 1 bis 6 Kohlenstoffatomen oder jeweils Phenyl sind.

4. Verfahren nach Anspruch 3, wobei R¹ = *tert*-Butyl; R² *= tert-*Butyl; und wobei R³ und R⁴ gleich sind und jeweils ausgewählt aus der Gruppe bestehend aus Methyl, Ethyl, *n-*Propyl, *iso*-Propyl, *n*-Butyl, *n*-Pentyl, *n*-Hexyl sind.

5. Verfahren nach einem der Ansprüche 1 bis 4, wobei **(A)** in **(C)** mindestens einem Lösungsmittel eingesetzt wird.

6. Verfahren nach einem der Ansprüche 1 bis 5, wobei **(B)** zusätzlich mit **(D)** vermischt wird, wobei **(D)** mindestens eine Verbindung ausgewählt aus der Gruppe bestehend aus Hydroxylaminen, Oximen, sterisch gehinderten *N*-Oxyl-Verbindungen, Chinonmethiden, Nitroaromaten, Nitrosoaromaten, Benzochinonen, Hydrochinonen, Phenothiazinen, phenolischen Antioxidantien, *N*,*N*'-Dialkyl-*p*-phenylendiaminen, *N*,*N*'-Dialkyl-*p*-benzochinondiimiden ist.

7. Zusammensetzung **(AB*)** umfassend
**(A)** mindestens eine Verbindung der chemischen Struktur **(I)** mit 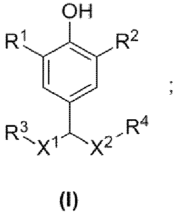
wobei X¹ und X² unabhängig voneinander jeweils S oder O ist;
wobei R¹, R² unabhängig voneinander jeweils ausgewählt aus der Gruppe bestehend aus Wasserstoff, Alkylgruppe mit 1 bis 18 Kohlenstoffatomen, Cycloalkylgruppe mit 3 bis 15 Kohlenstoffatomen, Arylgruppe mit 6 bis 14 Kohlenstoffatomen sind;
wobei R³, R⁴ unabhängig voneinander jeweils ausgewählt aus der Gruppe bestehend aus
-(CH₂)ₐ[O(CH₂)_{b}]_{c}R⁵, wobei R⁵ = H oder OH; a, c unabhängig voneinander jeweils eine ganze Zahl aus dem Bereich 1 bis 10 ist; und b = 1, 2, 3 oder 4 ist;
Alkylgruppe mit 1 bis 18 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR⁶, -OR⁷, -SR⁸, -NR⁹R¹⁰, -CN, Arylgruppe mit 6 bis 14 Kohlenstoffatomen substituiert sein kann;
Cycloalkylgruppe mit 3 bis 15 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹¹, -OR¹², -SR¹³, -NR¹⁴R¹⁵, -CN substituiert sein kann;
Arylgruppe mit 6 bis 14 Kohlenstoffatomen, bei welcher mindestens ein Wasserstoffrest durch einen Rest ausgewählt aus der Gruppe bestehend aus -F, -Cl, -Br, -I, -NH, -OH, -SH, -NHR¹⁶, -OR¹⁷, -SR¹⁸, -NR¹⁹R²⁰, -CN, Alkylgruppe mit 1 bis 6 Kohlenstoffatomen substituiert sein kann;
Heteroarylgruppe mit 1 bis 10 Kohlenstoffatomen, bei welcher mindestens ein an ein Kohlenstoffatom gebundener Wasserstoffrest durch einen Rest ausgewählt aus der Gruppe bestehend
aus -F, -Cl, -Br, -I, -NH, -OH, -SH, -NHR²¹, -OR²², -SR²³, -NR²⁴R²⁵, -CN, Alkylgruppe mit 1 bis 6 Kohlenstoffatomen substituiert sein kann;
Heterocycloalkylgruppe mit 2 bis 14 Kohlenstoffatomen, bei welcher mindestens ein an ein Kohlenstoffatom gebundener Wasserstoffrest durch einen Rest ausgewählt aus der Gruppe bestehend
aus -F, -Cl, -Br, -I, -NH, -OH, -SH, -NHR²⁶, -OR²⁷, -SR²⁸, -NR²⁹R³⁰, -CN substituiert sein kann;
wobei R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ unabhängig voneinander jeweils aus der Gruppe bestehend aus Alkylgruppe mit 1 bis 18 Kohlenstoffatomen, Cycloalkylgruppe mit 3 bis 15 Kohlenstoffatomen ausgewählt sind;
sind;
und
**(B)** mindestens ein olefinisch ungesättigtes Monomer ist,
**dadurch gekennzeichnet,**
**dass** das Gesamtgewicht aller von der Zusammensetzung **(AB*)** umfassten Verbindungen der Struktur **(I)** im Bereich von 1 ppm (m/m) bis 100.000 ppm (m/m) liegt, bezogen auf das Gesamtgewicht aller von der Zusammensetzung **(AB*)** umfassten olefinisch ungesättigten Monomere, und
wobei das olefinisch ungesättigte Monomer ausgewählt aus der Gruppe bestehend aus Ethen, Propen, Buten, 2-Methylpropen, 1,3-Butadien, 2-Methyl-1,3-Butadien, Vinylacetat, Acrylat, Methacrylat, Acrylsäure, Methacrylsäure, Acrolein, Acrylnitril, Styrol, Divinylbenzol, Cyclopentadien, Vinylchlorid, 1,3-Pentadien, Chloropren, *N*-Vinylformamid ist.

8. Zusammensetzung **(AB*)** nach Anspruch 7, wobei
X¹ = X₂ = O;
R¹, R² unabhängig voneinander jeweils ausgewählt aus der Gruppe bestehend aus Wasserstoff, Alkylgruppe mit 1 bis 18 Kohlenstoffatomen sind;
und R³, R⁴ unabhängig voneinander jeweils ausgewählt aus der Gruppe bestehend aus Alkylgruppe mit 1 bis 18 Kohlenstoffatomen, Arylgruppe mit 6 bis 14 Kohlenstoffatomen, Benzyl sind.

9. Zusammensetzung **(AB*)** nach Anspruch 8, wobei R¹ und R² gleich sind und jeweils ausgewählt aus der Gruppe bestehend aus Methyl, *n*-Propyl, *iso*-Propyl, *tert*-Butyl sind; und wobei R³ und R⁴ gleich sind und jeweils eine Alkylgruppe mit 1 bis 6 Kohlenstoffatomen oder jeweils Phenyl sind.

10. Zusammensetzung **(AB*)** nach Anspruch 9, wobei R¹ = *tert*-Butyl; R² = *tert*-Butyl; und wobei R³ und R⁴ gleich sind und jeweils ausgewählt aus der Gruppe bestehend aus Methyl, Ethyl, *n*-Propyl, *iso*-Propyl, *n*-Butyl, *n*-Pentyl, *n*-Hexyl sind.

11. Zusammensetzung **(AB*)** nach einem der Ansprüche 7 bis 10, zusätzlich umfassend **(C)** mindestens ein Lösungsmittel.

12. Zusammensetzung **(AB*)** nach einem der Ansprüche 7 bis 11, zusätzlich umfassend **(D)** mindestens eine Verbindung ausgewählt aus der Gruppe bestehend aus Hydroxylaminen, Oximen, sterisch gehinderten *N*-Oxyl-Verbindungen, Chinonmethiden, Nitroaromaten, Nitrosoaromaten, Benzochinonen, Hydrochinonen, Phenothiazinen, phenolischen Antioxidantien, *N*,*N*'-Dialkyl-*p*-phenylendiaminen, *N*,*N*'-Dialkyl-*p*-benzochinondiimiden.

## Claims

1. Process for inhibiting the polymerization of **(B)**, **characterized in that (A)** is mixed with **(B)** to obtain a composition **(AB)**, wherein
**(A)** is at least one compound having chemical structure **(I)** where 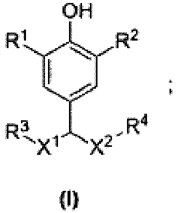
wherein X¹ and X² are each independently of one another S or O;
wherein R¹, R² are each independently of one another selected from the group consisting of hydrogen, alkyl group having 1 to 18 carbon atoms, cycloalkyl group having 3 to 15 carbon atoms, aryl group having 6 to 14 carbon atoms;
wherein R³, R⁴ are each independently of one another selected from the group consisting of
-(CH₂)ₐ[O(CH₂)_{b}]_{c}R⁵, wherein R⁵ = H or OH; a, c are each independently of one another an integer from the range 1 to 10; and b = 1, 2, 3 or 4;
alkyl group having 1 to 18 carbon atoms, where at least one hydrogen radical may be substituted by a radical selected from the group consisting of -F, -Cl, -Br, -I, -NH, -OH, -SH, -NHR⁶, -OR⁷, -SR⁸, -NR⁹R¹⁰, -CN, aryl group having 6 to 14 carbon atoms;
cycloalkyl group having 3 to 15 carbon atoms, where at least one hydrogen radical may be substituted by a radical selected from the group consisting of -F, -Cl, -Br, -I, -NH, -OH, -SH, -NHR¹¹, -OR¹², -SR¹³, -NR¹⁴R¹⁵, -CN;
aryl group having 6 to 14 carbon atoms, where at least one hydrogen radical may be substituted by a radical selected from the group consisting of -F, -Cl, -Br, -I, -NH, -OH, -SH, -NHR¹⁶, -OR¹⁷, -SR¹⁸, -NR¹⁹R²⁰, -CN, alkyl group having 1 to 6 carbon atoms;
heteroaryl group having 1 to 10 carbon atoms, where at least one hydrogen radical bonded to a carbon atom may be substituted by a radical selected from the group consisting of -F, -Cl, -Br, -I, -NH, -OH, -SH, -NHR²¹,-OR²², -SR²³, -NR²⁴R²⁵, -CN, alkyl group having 1 to 6 carbon atoms;
heterocycloalkyl group having 2 to 14 carbon atoms where at least one hydrogen radical bonded to a carbon atom may be substituted by a radical selected from the group consisting of -F, -Cl, -Br, -I, -NH, -OH, -SH, -NHR²⁶, -OR²⁷, -SR²⁸, -NR²⁹R³⁰, -CN;
wherein R⁶, R⁷, R⁸, R⁹, R¹⁰, R¹¹, R¹², R¹³, R¹⁴, R¹⁵, R¹⁶ R¹⁷, R¹⁸, R¹⁹, R²⁰, R²¹, R²², R²³, R²⁴, R²⁵, R²⁶, R²⁷, R²⁸, R²⁹, R³⁰ are each independently of one another selected from the group consisting of alkyl group having 1 to 18 carbon atoms, cycloalkyl group having 3 to 15 carbon atoms;
and wherein
**(B)** is at least one olefinically unsaturated monomer, wherein **(A)** is employed in an amount such that the total weight of all compounds of structure **(I)** employed in the process is in the range from 1 ppm (m/m) to 100 000 ppm (m/m) in each case based on the total weight of all olefinically unsaturated monomers employed in the process,
and wherein the olefinically unsaturated monomer is selected from the group consisting of ethene, propene, butene, 2-methylpropene, 1,3-butadiene, 2-methyl-1,3-butadiene, vinyl acetate, acrylate, methacrylate, acrylic acid, methacrylic acid, acrolein, acrylonitrile, styrene, divinylbenzene, cyclopentadiene, vinyl chloride, 1,3-pentadiene, chloroprene, N-vinylformamide.

2. Process according to Claim 1, wherein
X¹ = X² = O;
R¹, R² are each independently of one another selected from the group consisting of hydrogen, alkyl group having 1 to 18 carbon atoms;
and R³, R⁴ are each independently of one another selected from the group consisting of alkyl group having 1 to 18 carbon atoms, aryl group having 6 to 14 carbon atoms, benzyl.

3. Process according to Claim 2, wherein R¹ and R² are identical and are each selected from the group consisting of methyl, *n*-propyl, *iso*-propyl, *tert*-butyl; and wherein R³ and R⁴ are identical and are each an alkyl group having 1 to 6 carbon atoms or each phenyl.

4. Process according to Claim 3, wherein R¹ = *tert-*butyl; R² = *tert*-butyl; and wherein R³ and R⁴ are identical and are each selected from the group consisting of methyl, ethyl, *n*-propyl, *iso*-propyl, n-butyl, *n*-pentyl, *n*-hexyl.

5. Process according to any of Claims 1 to 4, wherein **(A)** is employed in **(C)** at least one solvent.

6. Process according to any of Claims 1 to 5, wherein **(B)** is additionally mixed with **(D)**, wherein **(D)** is at least one compound selected from the group consisting of hydroxylamines, oximes, sterically hindered N-oxyl compounds, quinone methides, nitroaromatics, nitrosoaromatics, benzoquinones, hydroquinones, phenothiazines, phenolic antioxidants, *N*,*N*'-dialkyl-*p-*phenylenediamines, *N*,*N*'-dialkyl-*p*-benzoquinonediimides.

7. Composition **(AB*)** comprising
**(A)** at least one compound of chemical structure **(I)** where 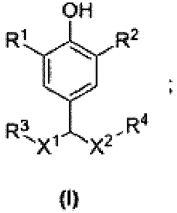
wherein X¹ and X² are each independently of one another S or O;
wherein R¹, R² are each independently of one another selected from the group consisting of hydrogen, alkyl group having 1 to 18 carbon atoms, cycloalkyl group having 3 to 15 carbon atoms, aryl group having 6 to 14 carbon atoms;
wherein R³, R⁴ are each independently of one another selected from the group consisting of
-(CH₂)ₐ[O(CH₂)_{b}]_{c}R⁵, wherein R⁵ = H or OH; a, c are each independently of one another an integer from the range 1 to 10; and b = 1, 2, 3 or 4;
alkyl group having 1 to 18 carbon atoms, where at least one hydrogen radical may be substituted by a radical selected from the group consisting of -F, -Cl, -Br, -I, -NH, -OH, -SH, -NHR⁶, -OR⁷, -SR⁸, -NR⁹R¹⁰, -CN, aryl group having 6 to 14 carbon atoms;
cycloalkyl group having 3 to 15 carbon atoms, where at least one hydrogen radical may be substituted by a radical selected from the group consisting of -F, -Cl, -Br, -I, -NH, -OH, -SH, -NHR¹¹, -OR¹², -SR¹³, -NR¹⁴R¹⁵, -CN;
aryl group having 6 to 14 carbon atoms, where at least one hydrogen radical may be substituted by a radical selected from the group consisting of -F, -Cl, -Br, -I, -NH, -OH, -SH, -NHR¹⁶, -OR¹⁷, -SR¹⁸, -NR¹⁹R²⁰, -CN, alkyl group having 1 to 6 carbon atoms;
heteroaryl group having 1 to 10 carbon atoms, where at least one hydrogen radical bonded to a carbon atom may be substituted by a radical selected from the group consisting of -F, -Cl, -Br, -I, -NH, -OH, -SH, -NHR²¹, -OR²², -SR²³, -NR²⁴R²⁵, -CN, alkyl group having 1 to 6 carbon atoms;
heterocycloalkyl group having 2 to 14 carbon atoms where at least one hydrogen radical bonded to a carbon atom may be substituted by a radical selected from the group consisting of -F, -Cl, -Br, -I, -NH, -OH, -SH, -NHR²⁶, -OR²⁷, -SR²⁸, -NR²⁹R³⁰, -CN;
wherein R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ are each independently of one another selected from the group consisting of alkyl group having 1 to 18 carbon atoms, cycloalkyl group having 3 to 15 carbon atoms;
and
**(B)** is at least one olefinically unsaturated monomer, **characterized in that**,
the total weight of all compounds of structure **(I)** comprised by the composition **(AB*)** is in the range from 1 ppm (m/m) to 100 000 ppm (m/m) based on the total weight of all olefinically unsaturated monomers comprised by the composition **(AB*),** and
wherein the olefinically unsaturated monomer is selected from the group consisting of ethene, propene, butene, 2-methylpropene, 1,3-butadiene, 2-methyl-1,3-butadiene, vinyl acetate, acrylate, methacrylate, acrylic acid, methacrylic acid, acrolein, acrylonitrile, styrene, divinylbenzene, cyclopentadiene, vinyl chloride, 1,3-pentadiene, chloroprene, N-vinylformamide.

8. Composition **(AB*)** according to Claim 7, wherein
X¹ = X² = O;
R¹, R² are each independently of one another selected from the group consisting of hydrogen, alkyl group having 1 to 18 carbon atoms;
and R³, R⁴ are each independently of one another selected from the group consisting of alkyl group having 1 to 18 carbon atoms, aryl group having 6 to 14 carbon atoms, benzyl.

9. Composition **(AB*)** according to Claim 8, wherein R¹ and R² are identical and are each selected from the group consisting of methyl, *n*-propyl, *iso*-propyl, *tert-*butyl; and wherein R³ and R⁴ are identical and are each an alkyl group having 1 to 6 carbon atoms or each phenyl.

10. Composition **(AB*)** according to Claim 9, wherein R¹ = *tert*-butyl; R² = *tert*-butyl; and wherein R³ and R⁴ are identical and are each selected from the group consisting of methyl, ethyl, *n*-propyl, *iso*-propyl, n-butyl, *n*-pentyl, *n*-hexyl.

11. Composition **(AB*)** according to any of Claims 7 to 10, further comprising **(C)** at least one solvent.

12. Composition **(AB*)** according to any of Claims 7 to 11, further comprising **(D)** at least one compound selected from the group consisting of hydroxylamines, oximes, sterically hindered *N*-oxyl compounds, quinone methides, nitroaromatics, nitrosoaromatics, benzoquinones, hydroquinones, phenothiazines, phenolic antioxidants, *N*,*N*'-dialkyl-*p*-phenylenediamines, *N,N'-*dialkyl-*p*-benzoquinonediimides.

## Revendications

1. Procédé pour l'inhibition de la polymérisation de **(B)**, **caractérisé en ce qu'**on mélange **(A)** avec **(B)** et on obtient ainsi une composition **(AB),** dans lequel
**(A)** est au moins un composé de structure chimique **(I)** suivante 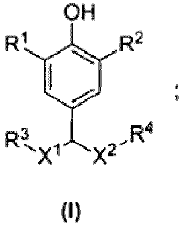
dans laquelle X¹ et X² représentent chacun indépendamment l'un de l'autre S ou O ;
dans laquelle R¹ et R² sont choisis chacun indépendamment l'un de l'autre dans le groupe constitué par un atome d'hydrogène, un groupe alkyle ayant de 1 à 18 atomes de carbone, un groupe cycloalkyle ayant de 3 à 15 atomes de carbone, un groupe aryle ayant de 6 à 14 atomes de carbone ;
dans laquelle R³ et R⁴ sont choisis chacun indépendamment l'un de l'autre dans le groupe constitué par
-(CH₂)ₐ[O(CH₂)_{b}]_{c}R⁵, R⁵ étant H ou OH ; a, c représentant chacun indépendamment l'un de l'autre un nombre entier choisi dans la plage de 1 à 10 ; et b valant 1, 2, 3 ou 4 ;
un groupe alkyle ayant de 1 à 18 atomes de carbone, dans lequel au moins un radical hydrogène peut être remplacé par un radical choisi dans le groupe constitué par
-F, -Cl, -Br, -I, -NH, -OH, -SH, -NHR⁶, -OR⁷, -SR⁸, -NR⁹R¹⁰, -CN, un groupe aryle ayant de 6 à 14 atomes de carbone ;
un groupe cycloalkyle ayant de 3 à 15 atomes de carbone, dans lequel au moins un radical hydrogène peut être remplacé par un radical choisi dans le groupe constitué par
-F, -Cl, -Br, -I, -NH, -OH, -SH, -NHR¹¹, -OR¹², -SR¹³, -NR¹⁴R¹⁵, -CN ;
un groupe aryle ayant de 6 à 14 atomes de carbone, dans lequel au moins un radical hydrogène peut être remplacé par un radical choisi dans le groupe constitué par -F, -Cl, -Br, -I, -NH, -OH, -SH, -NHR¹⁶, -OR¹⁷, -SR¹⁸, -NR¹⁹R²⁰, -CN, un groupe alkyle ayant de 1 à 6 atomes de carbone ;
un groupe hétéroaryle ayant de 1 à 10 atomes de carbone, dans lequel au moins un radical hydrogène lié à un atome de carbone peut être remplacé par un radical choisi dans le groupe constitué par -F, -Cl, -Br, -I, -NH, -OH, -SH, -NHR²¹, -OR²², -SR²³, -NR²⁴R²⁵, -CN, un groupe alkyle ayant de 1 à 6 atomes de carbone ;
un groupe hétérocycloalkyle ayant de 2 à 14 atomes de carbone, dans lequel au moins un radical hydrogène lié à un atome de carbone peut être remplacé par un radical choisi dans le groupe constitué par -F, -Cl, -Br, -I, -NH, -OH, -SH, -NHR²⁶, -OR²⁷, -SR²⁸, -NR²⁹R³⁰, -CN ;
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ étant choisis chacun indépendamment les uns des autres dans le groupe constitué par un groupe alkyle ayant de 1 à 18 atomes de carbone, un groupe cycloalkyle ayant de 3 à 15 atomes de carbone ;
et dans lequel
**(B)** est au moins un monomère à insaturation oléfinique, dans lequel on utilise **(A)** en une quantité telle que le poids total de tous les composés de structure **(I)** utilisés dans le procédé se situe dans la plage de 1 ppm (m/m) à 100 000 ppm (m/m), chaque fois par rapport au poids total de tous les monomères à insaturation oléfinique utilisés dans le procédé,
et dans lequel le monomère à insaturation oléfinique est choisi dans le groupe constitué par l'éthène, le propène, le butène, le 2-méthylpropène, le 1,3-butadiène, le 2-méthyl-1,3-butadiène, l'acétate de vinyle, un acrylate, un méthacrylate, l'acide acrylique, l'acide méthacrylique, l'acroléine, l'acrylonitrile, le styrène, le divinylbenzène, le cyclopentadiène, le chlorure de vinyle, le 1,3-pentadiène, le chloroprène, le N-vinylformamide.

2. Procédé selon la revendication 1, dans lequel X¹ = X² = O ;
R¹, R² sont choisis chacun indépendamment l'un de l'autre dans le groupe constitué par un atome d'hydrogène, un groupe alkyle ayant de 1 à 18 atomes de carbone ;
et R³, R⁴ sont choisis chacun indépendamment l'un de l'autre dans le groupe constitué par un groupe alkyle ayant de 1 à 18 atomes de carbone, un groupe aryle ayant de 6 à 14 atomes de carbone, le groupe benzyle.

3. Procédé selon la revendication 2, dans lequel R¹ et R² sont identiques et sont choisis chacun dans le groupe constitué par méthyle, *n*-propyle, *iso*-propyle, *tert*-butyle ; et dans lequel R³ et R⁴ sont identiques et représentent chacun un groupe alkyle ayant de 1 à 6 atomes de carbone ou chacun le groupe phényle.

4. Procédé selon la revendication 3, dans lequel R¹ = *tert*-butyle ; R² = *tert*-butyle ; et dans lequel R³ et R⁴ sont identiques et sont choisis chacun dans le groupe constitué par méthyle, éthyle, *n*-propyle, iso-propyle, *n*-butyle, *n*-pentyle, *n*-hexyle.

5. Procédé selon l'une quelconque des revendications 1 à 4, dans lequel **(A)** est utilisé dans **(C)** au moins un solvant.

6. Procédé selon l'une quelconque des revendications 1 à 5, dans lequel en outre on mélange **(B)** avec **(D), (D)** étant au moins un composé choisi dans le groupe constitué par des hydroxylamines, des oximes, des composés N-oxyle à encombrement stérique, des quinoneméthides, des composés aromatiques nitrés, des composés nitrosoaromatiques, des benzoquinones, des hydroquinones, des phénothiazines, des antioxydants phénoliques, des *N*,*N*'-dialkyl-*p*-phénylènediamines, des *N*,*N*'-dialkyl-*p*-benzoquinonediimides.

7. Composition **(AB*)** comprenant
**(A)** au moins un composé de structure chimique **(I)** suivante 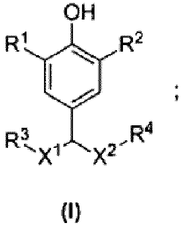
dans laquelle X¹ et X² représentent chacun indépendamment l'un de l'autre S ou O ;
dans laquelle R¹ et R² sont choisis chacun indépendamment l'un de l'autre dans le groupe constitué par un atome d'hydrogène, un groupe alkyle ayant de 1 à 18 atomes de carbone, un groupe cycloalkyle ayant de 3 à 15 atomes de carbone, un groupe aryle ayant de 6 à 14 atomes de carbone ;
dans laquelle R³ et R⁴ sont choisis chacun indépendamment l'un de l'autre dans le groupe constitué par
-(CH₂)ₐ[O(CH₂)_{b}]_{c}R⁵, R⁵ étant H ou OH ; a, c représentant chacun indépendamment l'un de l'autre un nombre entier choisi dans la plage de 1 à 10 ; et b valant 1, 2, 3 ou 4 ;
un groupe alkyle ayant de 1 à 18 atomes de carbone, dans lequel au moins un radical hydrogène peut être remplacé par un radical choisi dans le groupe constitué par
-F, -Cl, -Br, -I, -NH, -OH, -SH, -NHR⁶, -OR⁷, -SR⁸, -NR⁹R¹⁰, -CN, un groupe aryle ayant de 6 à 14 atomes de carbone ;
un groupe cycloalkyle ayant de 3 à 15 atomes de carbone, dans lequel au moins un radical hydrogène peut être remplacé par un radical choisi dans le groupe constitué par
-F, -Cl, -Br, -I, -NH, -OH, -SH, -NHR¹¹, -OR¹², -SR¹³, -NR¹⁴R¹⁵, -CN ;
un groupe aryle ayant de 6 à 14 atomes de carbone, dans lequel au moins un radical hydrogène peut être remplacé par un radical choisi dans le groupe constitué par -F, -Cl, -Br, -I, -NH, -OH, -SH, -NHR¹⁶, -OR¹⁷, -SR¹⁸, -NR¹⁹R²⁰, -CN, un groupe alkyle ayant de 1 à 6 atomes de carbone ;
un groupe hétéroaryle ayant de 1 à 10 atomes de carbone, dans lequel au moins un radical hydrogène lié à un atome de carbone peut être remplacé par un radical choisi dans le groupe constitué par -F, -Cl, -Br, -I, -NH, -OH, -SH, -NHR²¹, -OR²², -SR²³, -NR²⁴R²⁵, -CN, un groupe alkyle ayant de 1 à 6 atomes de carbone ;
un groupe hétérocycloalkyle ayant de 2 à 14 atomes de carbone, dans lequel au moins un radical hydrogène lié à un atome de carbone peut être remplacé par un radical choisi dans le groupe constitué par -F, -Cl, -Br, -I, -NH, -OH, -SH, -NHR²⁶, -OR²⁷, -SR²⁸, -NR²⁹R³⁰, -CN ;
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ étant choisis chacun indépendamment les uns des autres dans le groupe constitué par un groupe alkyle ayant de 1 à 18 atomes de carbone, un groupe cycloalkyle ayant de 3 à 15 atomes de carbone ;
et
**(B)** au moins un monomère à insaturation oléfinique, **caractérisée en ce que**
le poids total de tous les composés de structure **(I)** englobés par la composition **(AB*)** se situe dans la plage de 1 ppm (m/m) à 100 000 ppm (m/m), par rapport au poids total de tous les monomères à insaturation oléfinique englobés par la composition **(AB*),** et
le monomère à insaturation oléfinique étant choisi dans le groupe constitué par l'éthène, le propène, le butène, le 2-méthylpropène, le 1,3-butadiène, le 2-méthyl-1,3-butadiène, l'acétate de vinyle, un acrylate, un méthacrylate, l'acide acrylique, l'acide méthacrylique, l'acroléine, l'acrylonitrile, le styrène, le divinylbenzène, le cyclopentadiène, le chlorure de vinyle, le 1,3-pentadiène, le chloroprène, le N-vinylformamide.

8. Composition **(AB*)** selon l'invention 7, dans laquelle
X¹ = X² = O ;
R¹, R² sont choisis chacun indépendamment l'un de l'autre dans le groupe constitué par un atome d'hydrogène, un groupe alkyle ayant de 1 à 18 atomes de carbone ;
et R³, R⁴ sont choisis chacun indépendamment l'un de l'autre dans le groupe constitué par un groupe alkyle ayant de 1 à 18 atomes de carbone, un groupe aryle ayant de 6 à 14 atomes de carbone, le groupe benzyle.

9. Composition **(AB*)** selon l'invention 8, dans laquelle R¹ et R² sont identiques et sont choisis chacun dans le groupe constitué par méthyle, *n*-propyle, *iso-*propyle, *tert*-butyle ; et dans laquelle R³ et R⁴ sont identiques et représentent chacun un groupe alkyle ayant de 1 à 6 atomes de carbone ou chacun le groupe phényle.

10. Composition **(AB*)** selon la revendication 9, dans laquelle R¹ = *tert*-butyle ; R² = *tert*-butyle ; et dans laquelle R³ et R⁴ sont identiques et sont choisis chacun dans le groupe constitué par méthyle, éthyle, *n*-propyle, *iso*-propyle, *n*-butyle, *n*-pentyle, *n*-hexyle.

11. Composition **(AB*)** selon l'une quelconque des revendications 7 à 10, comprenant en outre **(C)** au moins un solvant.

12. Composition **(AB*)** selon l'une quelconque des revendications 7 à 11, comprenant en outre **(D)** au moins un composé choisi dans le groupe constitué par des hydroxylamines, des oximes, des composés N-oxyle à encombrement stérique, des quinoneméthides, des composés aromatiques nitrés, des composés nitrosoaromatiques, des benzoquinones, des hydroquinones, des phénothiazines, des antioxydants phénoliques, des *N*,*N*'-dialkyl-*p*-phénylènediamines, des *N*,*N*'-dialkyl-*p*-benzoquinonediimides.
